# EUROPEAN PATENT APPLICATION

(11) **EP 3 637 424 A1**
(43) Date of publication of application: **15.04.2020**
(21) Application number: 18199915.2
(22) Date of filing: 11.10.2018
(51) Int. Cl.: G16H 10/60

(54) **HEALTHCARE NETWORK**

(71) Applicant: Siemens Healthcare GmbH, 91052 Erlangen (DE)
(72) Inventor: Deckert, Dirk - André, 81539 München (DE); Fernández-Gutiérrez, Fabiola, 91052 Erlangen (DE); Jattke, Kirstin, 90482 Nürnberg (DE); Nikonov, Andrej, 80639 München (DE); Roth, Dorothee, 91054 Erlangen (DE); Unger, Milan, 930 41 Kvetoslavov (SK)

(57) **Abstract**

A system operable to transmit healthcare data to a user device configured for use in analysing medical information. A directed graph representing at least one medical guideline is maintained in a database. The directed graph comprising a plurality of nodes connected by a plurality of directed edges. In some examples a directed graph is selected based on a medical condition and a context parameter. In some examples a directed graph is generated based on differences between versions of medical guidelines. A patient model may be maintained in a database and in some examples based on a combination of the directed graph and the patient model, further healthcare data is transmitted to the user device. The status of nodes and edges may be determined based on a combination of a directed graph and a patient model.

## Description

Embodiments described herein relate generally to providing healthcare data to a user device. More specifically the embodiments relate to methods, systems, and computer programs for transmitting healthcare data to a user device configured for use in analysing medical information.

Medical guidelines provide recommendations for how people with specific medical conditions should be treated. Medical guidelines may indicate which diagnostic or therapeutic steps should be taken when treating a patient with a specific condition and what follow-up procedures should be performed dependent on the results of the diagnostic or therapeutic steps. Some medical guidelines provide information about the prevention, prognosis of certain medical conditions as well as the risk and/or benefits, and take in account the cost-effectiveness associated with diagnostic and therapeutic steps in the treatment of a patient. The information contained within a guideline is generally specific to a particular medical domain.

Data pertaining to patients being treated for a medical condition are typically generated during diagnostic and therapeutic steps. This data is typically stored in disparate sources relating to the locations, such as clinical centres or hospitals, in which the data is generated. Data pertaining to patients may be encoded to relate the raw data or values with the respective clinical steps which generated said data. Data may be encoded using clinical coding systems such as SNOMED CT, LOINC, Siemens® internal coding system, among other coding systems.

Patient conditions and diseases do not always conform with recommendations and clinical pathways provided in Medical guidelines. Medical guidelines are regularly updated to reflect changes and improvements in clinical pathways for diseases. A clinical pathway, also called a disease pathway, may include secondary prevention, screening, diagnostics, diagnosis, therapy decisions, therapy and follow-up treatments or decisions. As such, a medical guideline alone may not always be sufficient to enable sufficient analysis.

Therefore, it is desirable to improve ease and accessibility of medical information that is transmitted to a user device for analysis.

A system for generating a decision graph based on medical guidelines is described in US 2016/0321402 A1, wherein decision graphs reflecting the logic encoded in the medical guideline are evaluated with respect to patient records.

### Summary

In a first embodiment, there is provided a system operable to transmit healthcare data to a user device the user device being configured for use in analysing medical information, the system comprising at least one processor and at least one memory including computer program code, the at least one memory and computer program code configured to, with the at least one processor, cause the system to perform the steps of:
maintaining, in a database, data representing a first directed graph representing at least part of a first version of a medical guideline, the first directed graph comprising a first plurality of nodes and a first set of directed edges, each node of the first plurality of nodes being connected to at least one further node of the first plurality of nodes by one of the first set of directed edges, the first directed graph comprising a primary node and terminating in at least one end node;
responsive to a determination that a second version of the medical guideline is available, generating a first set of associations, each being between one of a second, different plurality of nodes of a directed graph that may represent at least part of the second version and a respective part of the second version;
identifying, based on the first set of associations, one or more differences between the directed graph that may represent at least part of the second version and the first directed graph;
generating a second directed graph based at least one the one or more differences and the first directed graph; and
transmitting data representing the second directed graph for receipt by the user device.

The healthcare data (also referred to as medical or clinical data and/or information) may comprise data related to a medical guideline such as text comprised in a medical guideline and/or directed graphs representing medical guidelines. Healthcare data also comprises secondary information relating to medical conditions such as research papers, results from clinical trials, data collected from patients in the course of ongoing treatment of medical conditions. Healthcare data also comprises data relating to specific patients collected during clinical steps. Healthcare data may comprise electronic records of one or more patients, patient and/or disease registries comprising data relating to chronic conditions.

Therefore, the user device may be provided with an updated version of at least part of a medical guideline in the form of a directed graph to use when analysing medical information relating to a patient being treated for a medical condition associated with the medical guideline.

The system may determine that an updated version of the medical guideline is available by comparing metadata associated with the first medical guideline with meta data associated with one or more further, different, medical guidelines stored at one or more medical guideline repositories. Thereby the system may automatically determine that an updated version of the medical guideline is available at a medical guideline repository. The medical guideline repository may comprise all medical guidelines relevant to a particular medical condition, or to a particular country or practice group.

In examples, each of the first plurality of nodes represents a clinical step. In some examples each of the first set of directed edges represents a conditional parameter value resulting from a said clinical step associated with one of the first plurality of nodes connected thereto. Thereby a user of the user device may review an updated directed graph comprising indications of each of the clinical steps to be performed during the treatment of a patient with a particular medical condition and what conditions must be satisfied when moving from one clinical step to a next clinical step.

The system may additionally: generate a second, different, set of associations, each being between one of a second, different, set of directed edges of the directed graph that may represent at least part of the second version and a respective part of the second version; identify, based on the second set of associations, one or more further differences between the second set of directed edges and the first set of directed edges; and generate the second directed graph dependent at least on the one or more further differences and the first directed graph.

Thereby, the system may update the directed graph based on differences in the conditional parameter values which determine when a patient may move from one clinical step to another clinical step or in some cases the pathway a patient may take between different clinical steps described in the medical guideline may change.

The system may additionally: responsive to the determination compare a first textual excerpt of the first version of the medical guideline to a corresponding second textual excerpt of the second version of the medical guideline; and transmit data representing a result of the comparison for receipt by the user device.

Thereby healthcare data in the form of textual excerpts from medical guidelines may be updated and relevant medical information may be transmitted to the user device.

Generating the second directed graph may comprise: based on a user input indicative of a decision in respect of at least one of the one or more differences, selectively using at least part of the first version or the second version to generate a respective part of the second directed graph.

The first directed graph may comprise data indicative of at least one local modification made by a user of the system. This allows the guidelines used in treating patients to be customised, for example, to cater to patient requirements and/or policies.

In an example, a part of the second directed graph may be based on the at least one local modification.

The system may additionally: maintain, in a database, a patient model comprising healthcare data associated with a patient; and determine, based on the combination of the first directed graph and the patient model, a first clinical pathway represented by at least some of the first plurality of nodes and at least one of the first set of directed edges. The healthcare data comprised in the patient model may comprise test results from diagnostic tests or therapeutic treatments performed on the patient. Thereby the system enables monitoring of a clinical pathway associated with a patient.

Generating the second directed graph may comprise determining, based on a combination of the first clinical pathway and the one or more identified differences, a second clinical pathway represented by at least some of a third, different, plurality of nodes comprised in the second directed graph and at least some of a third, different, set of directed edges comprised in the second directed graph. This may enable the status of the patient to be identified in accordance with the updated version of the medical guideline. This allows the first clinical pathway of the patient to be mapped to the second directed graph.

Determining the second clinical pathway may comprise transmitting data indicative of a comparison between the first clinical pathway and the one or more differences for receipt by the user device; receiving, from the user device, data indicating a command; and determining, the second clinical pathway based at least on the received command. Thereby a user of the user device, such as a medical practitioner, may be able to review differences which alter the mapped status of the patient along the first clinical pathway and manually modify the second clinical pathway e.g. override one or more of the consequential changes in the second clinical pathway.

In a second embodiment, there is provided a method of transmitting healthcare data to a user device, the user device being configured for use in analysing medical information, the method comprising:
maintaining, in a database, data representing a first directed graph representing at least part of a first version of a medical guideline, the first directed graph comprising a first plurality of nodes and a first set of directed edges, each node of the plurality of nodes being connected to at least one further node of the plurality of nodes by one of the first set of directed edges, the first directed graph comprising a primary node and terminating in at least one end node;
responsive to a determination that a second version of the medical guideline is available, generating a first set of associations, each being between one of a second, different plurality of nodes of a directed graph that may represent at least part of the second version and a respective part of the second version;
identifying, based on the first set of associations, one or more differences between the directed graph that may represent at least part of the second version and the first directed graph;
generating a second directed graph based at least on the one or more differences and the first directed graph,
transmitting data representing the second directed graph for receipt by the user device.

In a third embodiment, there is provided a computer program comprising a set of instructions, which, when executed by a computerised device, cause the computerised device to perform a method of transmitting healthcare data to a user device, the user device being configured for use in analysing medical information, the method comprising, at the computerised device:
maintaining, in a database, data representing a first directed graph representing at least part of a first version of a medical guideline, the first directed graph comprising a first plurality of nodes and a first set of directed edges, each node of the plurality of nodes being connected to at least one further node of the plurality of nodes by one of the first set of directed edges, the first directed graph comprising a primary node and terminating in at least one end node;
responsive to a determination that a second version of the medical guideline is available, generating a first set of associations, each being between one of a second, different, plurality of nodes of a directed graph that may represent at least part of the second version and a respective part of the second version;
identifying, based on the first set of associations, one or more differences between the directed graph that may represent at least part of the second version and the first directed graph; and
generating a second directed graph based at least on the one or more differences and the first directed graph; and
transmitting data representing the second directed graph for receipt by the user device.

In a fourth embodiment, there is provided a system operable to transmit healthcare data to a user device, the user device being configured for use in analysing medical information, the system comprising at least one processor and at least one memory including computer program code, the at least one memory and the computer program code configured to, with the at least one processor, cause the system to perform the steps of:
maintaining, in a database, a plurality of directed graphs, each directed graph representing at least part of at least one medical guideline and comprising a plurality of nodes and a set of directed edges, each node of the plurality of nodes being connected to at least one further node of the plurality of nodes by one of the set of directed edges, each directed graph comprising a primary node and terminating in at least one end node;
receiving, from the user device, healthcare data comprising data identifying a medical condition;
determining a context parameter based on the received healthcare data and an identifier of the user device;
selecting, based on the determined context parameter and the data identifying the medical condition, a directed graph from the plurality of directed graphs; and
transmitting data indicative of the selected directed graph for receipt by the user device.

This enables selection of a directed graph based on a medical condition and a context parameter, which allows a more suitable and/or tailored medical guideline to be provided to the user device when analysing medical information, for example, associated with a patient.

In an example, each of the nodes represents a clinical step. In an example, each of the directed edges represents a conditional parameter value resulting from a said clinical step associated with one of the nodes connected thereto.

The system may additionally maintain, in a database, a plurality of patient models each comprising healthcare data associated with a respective patient.

Thereby the system may centralise healthcare data, such as test results, relating to a patient collected from a plurality of disparate sources.

The context parameter may comprise data indicative of one of the plurality of patient models. This allows the selected graph which is chosen to be specific to a particular patient, and thereby the selected directed graph may be configured to provide tailored healthcare to a particular patient based on, for example, the patient's preferences or needs.

The context parameter may comprise data associated with at least one patient entry. This may enable selection of a patient model and thereby a directed graph using a patient entry without requiring identification of a patient. For example, a test result from a clinical step performed on the patient may be used to identify the patient and thereby the directed graph to be selected.

The system may additionally: select, based on the context parameter and the data identifying the medical condition, a further directed graph from the plurality of directed graphs; and transmit data indicative of the selected further directed graph for receipt by the user device. Thereby, in cases wherein two directed graphs may be suitable for use in analysing medical information associated with a patient, both directed graphs may be transmitted to the user device for review.

The healthcare data comprising data identifying a medical condition received from the user device may be received via a network interface over a wide area network. Thereby, the user device may remotely access the system to select a directed graph.

The context parameter may comprise an indication of a location of the user device and/or an indication of a medical practitioner using the user device. Thereby the system may select a directed graph which is specific to the location of the user device, for example, a particular hospital having a specific medical guideline for treating a disease, or specific to the user of the user device, for example, a medical practitioner running a clinical trial in which a tailored medical guideline is needed.

The selected directed graph may comprise data indicative of a local modification of at least one of the nodes and/or at least one of the set of directed edges of the selected directed graph. This allows the selected directed graph to help provide tailored care to a patient using modifications by a user of the system.

The system may additionally: maintain, in a database, a plurality of textual excerpts from at the least one medical guideline; select, based on the medical guideline represented by the selected directed graph, at least one textual excerpt from the plurality of textual excerpts; and transmit data indicative of the textual excerpt for receipt by the user device. This allows the system to provide relevant healthcare data in the form of excerpts from a medical guideline to a user device when the user device is used in analysing medical information associated with a patient.

The system may transmit data to the user device over a wide area network via network interface. This allows the user device to receive relevant healthcare data when the user device is remote from the system such as when the system is located at a different hospital to the user device, or when the user device is being used remotely from a medical centre.

In a fifth embodiment, there is provided a method of transmitting healthcare data to a user device, the user device being configured for use in analysing medical information, the method comprising:
maintaining, in a database, a plurality of directed graphs, each directed graph representing at least part of at least one medical guideline and comprising a plurality of nodes and a set of directed edges, each node of the plurality of nodes being connected to at least one further node of the plurality of nodes by one of the set of directed edges, each directed graph comprising a primary node and terminating in at least one end node;
receiving, from the user device, healthcare data comprising data identifying a medical condition;
determining a context parameter based on the received healthcare data and an identifier of the user device;
selecting, based on the determined parameter and the data identifying the medical condition, a directed graph from the plurality of directed graphs; and
transmitting data indicative of the selected directed graph for receipt by the user device.

In a sixth embodiment, there is provided a computer program comprising a set of instructions, which, when executed by a computerised device, cause the computerised device to perform a method of transmitting healthcare data to a user device, the user device being configured for use in analysing medical information, the method comprising, at the computerised device:
maintaining, in a database, a plurality of directed graphs, each directed graph representing at least part of at least one medical guideline and comprising a plurality of nodes and a set of directed edges, each node of the plurality of nodes being connected to at least one further node of the plurality of nodes by one of the set of directed edges, each directed graph comprising a primary node and terminating in at least one end node;
receiving, from the user device, healthcare data comprising data identifying a medical condition;
determining a context parameter based on the received healthcare data and an identifier of the user device;
selecting, based on the determined parameter and the data identifying the medical condition, a directed graph from the plurality of directed graphs; and
transmitting data indicative of the selected directed graph for receipt by the user device.

In a seventh embodiment, there is provided a system operable to transmit healthcare data to a user device, the user device being configured for use in analysing medical information, the system comprising at least one processor and at least one memory including computer program code configured to, with the at least one processor, cause the system at least to perform the steps of:
maintaining, in a database, data representing a plurality of directed graphs, each directed graph representing at least part of at least one medical guideline and comprising a plurality of nodes and a set of directed edges, each node of the plurality of nodes being connected to at least one further node of the plurality of nodes by one of the set of directed edges, each directed graph comprising a primary node and terminating in at least one end node;
maintaining, in a database, a plurality of patient models each comprising healthcare data associated with a respective patient;
receiving, from the user device, healthcare data comprising data identifying a patient and a medical condition;
selecting, based at least on the received data identifying the medical condition, a directed graph from the plurality of directed graphs;
selecting, based on the received data identifying the patient, a patient model from the plurality of patient models;
dependent on a combination of the selected directed graph and the selected patient model, retrieving further healthcare data; and
transmitting the further healthcare data for receipt by the user device.

Thereby the system may be configured to automatically retrieve relevant healthcare data, dependent on the combination of the selected patient model and the selected directed graph. This allows relevant and helpful healthcare data to be transmitted to the user device to be displayed on the user device when a medical practitioner is analysing medical information, for example, test results. Thereby the user device may be provided with explicit instructions and/or medically relevant information for making decisions relating to a patient.

Further healthcare data may comprise at least one of: a textual excerpt from a said medical guideline associated with the selected directed graph; statistical information relating to a patient cohort associated with the selected patient model; a medical research paper; and consensus based supplementary medical data. Thereby the user device may be provided with supplementary information relevant to a specific context.

In some examples, each of the nodes represents a clinical step. In some examples, each of the directed edges of the selected directed graph represents a conditional parameter value resulting from a said clinical step associated with one of the nodes connected thereto.

The system may additionally maintain, in a database, at least one association between the further healthcare data and at least one directed graph, and wherein the further healthcare data is retrieved according to the at least one association. Thereby the further healthcare data which is retrieved is relevant to the medical condition associated with the directed graph at the user device.

The at least one association may be between the further healthcare data and a said node, and wherein the further healthcare data is retrieved according to the at least one association. This allows the further healthcare data to be more specifically relevant to a particular clinical step being reviewed at the user device when analysing medical information.

Retrieving the further healthcare data may be dependent on a status of at least one of the plurality of nodes and/or at least one of the set of directed edges of the selected directed graph.

The status of a said node is dependent upon availability of data associated with a clinical step represented by the node. The status of a said directed edge is dependent upon a combination of data associated with a clinical step represented by a said node connected thereto and a conditional parameter value represented by the directed edge. Thereby, the further healthcare data may be retrieved dependent on the status of the patient. For example, further healthcare data may be retrieved if the patient has failed a test and the user of the user device needs more information to determine the next steps for the patient.

The patient model may comprise a plurality of patient entries and wherein determining a status of a said node and a said directed edge connected thereto comprises the steps of:
maintaining a first association between at least one of the patient entries and an identifier from a plurality of identifiers;
maintaining a second association between at least one of the nodes and an identifier from the plurality of identifier;
selecting, based on the first and second associations, a said attribute value associated with the node; and
determining, based on a comparison of the attribute value associated with the node to a conditional parameter value represented by the directed edge, whether the conditional parameter value represented by the directed edge is satisfied.

Retrieving further healthcare data may be dependent on a determination that a further, different, said directed graph based on the selected patient model does not correspond with the selected directed graph. Thereby, the system may provide a user of the user device with supplementary information for improving healthcare provided to a patient even when the patient does not conform to standard medical guidelines.

The system may retrieve further healthcare data dependent on a determination that the status of at least one of the nodes and/or at least one of the set of directed edges of the selected directed graph cannot be determined based on the selected patient model. This may allow the system to retrieve further healthcare data to be used in determining the status of the at least one node or at least one directed edge which cannot be determined based on the plurality of attribute values.

The system may additionally: maintain, in a database, an association between a plurality of state parameters and the further healthcare data, the state parameters being for use in processing a decision at a said node of a directed graph; receive, from the user device, data indicating a rating associated with the retrieved further healthcare data; and process the rating to modify a said state parameter based on the received rating. The state parameters may be used to determine the suitability of further healthcare data retrieved when analysing medical information such as a particular node and or directed edge. Thereby, the system may allow a user to rate the suitability of further healthcare data to particular situations.

In an eighth embodiment, there is provided a computer program comprising a set of instructions, which, when executed by a computerised device, cause the computerised device to perform a method of transmitting healthcare data to a user device, the user device being configured for use in analysing medical information, the method comprising, at the computerised device:
maintaining, in a database, data representing a plurality of directed graphs, each directed graph representing at least part of least one medical guideline and comprising a plurality of nodes and a set of directed edges, each node of the plurality of nodes being connected to at least one further node of the plurality of nodes by one of the set of directed edges, each directed graph comprising a primary node and terminating in at least one end node;
maintaining, in a database, a plurality of patient models each comprising healthcare data associated with a respective patient;
receiving, from the user device, healthcare data comprising data identifying a patient and a medical condition;
selecting, based at least on the received data identifying the medical condition, a directed graph from the plurality of directed graphs;
selecting, based on the received data identifying the patient, a patient model from the plurality of patient models;
dependent on a combination of the selected directed graph and the selected patient model, retrieving further healthcare data; and
transmitting the further healthcare data for receipt by the user device.

In a ninth embodiment, there is provided a method of transmitting healthcare data to a user device, the user device being configured for use in analysing medical information associated with a patient, the method comprising:
maintaining, in a database, data representing a plurality of directed graphs, each directed graph representing at least part of at least one medical guideline and comprising a plurality of nodes and a set of directed edges, each node of the plurality of nodes being connected to at least one further node of the plurality of nodes by one of the set of directed edges, each directed graph comprising a primary node and terminating in at least one end node;
maintaining, in a database, a plurality of patient models each comprising healthcare data associated with a respective patient;
receiving, from the user device, healthcare data comprising data identifying a patient and a medical condition;
selecting, based at least on the received data identifying the medical condition, a directed graph from the plurality of directed graph;
selecting, based on the received data identifying the patient, a patient model from the plurality of patient models;
dependent on a combination of the selected directed graph and the selected patient model, retrieving further healthcare data; and
transmitting the further healthcare data for receipt by the user device.

In a tenth embodiment, there is provided a system operable to transmit healthcare data to a user device, the user device being configured for use in analysing medical information, the system comprising at least one processor and at least one memory including computer program code, the at least one memory and computer program code configured to, with the at least one processor, cause the system to perform the steps of:
maintaining, in a database, data representing a plurality of directed graphs, each directed graph representing at least part of at least one medical guideline and comprising a plurality of nodes and a set of directed edges, each node of the plurality of nodes being connected to at least one further node of the plurality of nodes by one of the set of directed edges, each directed graph comprising a primary node and terminating in at least one end node;
maintaining, in a database, a plurality of patient models each comprising healthcare data associated with a respective patient;
receiving, from the user device, healthcare data comprising data identifying a patient and a medical condition;
selecting, based at least on the received data identifying the medical condition, a directed graph from the plurality of directed graphs;
selecting, based on the received data identifying the patient, a patient model from the plurality of patient models;
identifying, based on the selected patient model and the received healthcare data, a status of at least one of the nodes and at least one of the set of directed edges of the selected directed graph; and
transmitting data associated with the status of the at least one of the nodes and the status of the at least one of the set of directed edges for receipt by the user device.

As discussed above, determining a status of at least one node and at least one directed edge of the selected directed graph based on the selected patient model allows the status of the patient to be determined such that a user of the user device is provided with an overview of the clinical steps a patient has undergone, and the current status of the patient, which may allow the user to determine best practice future clinical steps to be performed on a patient. Further, the determined status may allow the user to see how to conform to the medical guideline the patient, represented by the patient model, has been treated in the past and whether results from clinical steps performed on the patient deviate from the medical guideline.

In some examples, each of the nodes represents a clinical step. In some examples, each of the directed edges represents a conditional parameter value resulting from a said clinical step associated with one of the nodes connected thereto.

The status of a said node may be dependent upon availability of data associated with a clinical step represented by the node.

The status of a said directed edge may be dependent upon a combination of data associated with a clinical step represented by a said node connected thereto and a conditional parameter value represented by the directed edge.

In examples the selected patient model may comprise a plurality of patient entries and determining a status of a said node and a said directed edge connected thereto may comprise the steps of:
maintaining a first association between at least one of the patient entries and an identifier from a plurality of identifiers;
maintaining a second association between at least one of the nodes and an identifier from the plurality of identifiers;
selecting, based on the first and second associations, a said attribute value associated with the node; and
determining, based on a comparison of the attribute value associated with the node to the conditional parameter value represented by the directed edge, whether the conditional parameter value represented by the directed edge is satisfied.

The system may additionally: determine, based on an identified status of at least one of the nodes and at least one of the directed edges of the selected directed graph, such as by the examples described above, whether a further, different, said directed graph based on the selected patient model corresponds with the selected directed graph; and dependent on the determination, transmit data indicating the determination for receipt by the user device.

The system may additionally: determine, based on the identified status of the at least one of the nodes and the at least one directed edge, a start date and an end date of at least one treatment phase associated with the at least one medical guideline; and transmit data indicating the start and the end date of the at least on treatment phase for receipt by the user device. This may a determination of which treatment phase or phases the patient, represented by the selected patient model, has completed or is undergoing even if the patient model does not comprise a patient entry for each respective node and/or directed edge in the treatment phase. The treatment phase may be represented in the selected directed graph by at least some of the plurality of nodes connected by at least one of the set of directed edges. This allows a user to easily determine if a patient has deviated from the medical guideline and whether they need extra medical attention, for example, whether a treatment is not working and whether more information is need on and/or more clinical tests need to be performed on the patient.

The system may also retrieve further healthcare data associated with the at least one treatment phase; and transmit the further healthcare data associated with the at least one treatment phase for receipt by the user device. This allows the user of the user device to review information relevant to a treatment phase the patient has undergone or is currently undergoing to provide improved medical guidance and healthcare to the patient, for example, further healthcare data may indicate side effects associated with a treatment phase the patient is undergoing such that a user of the user device is able to inform the patient of the side effects associated with the current treatment phase.

The system may additionally identify, for at least on treatment phase, at least one node and at least one directed edge for which an attribute value cannot be selected based on the identifier; and determine the status of the at least one node and the at least one directed edge using the selected patient model and further healthcare data. By retrieving further healthcare data, for example, patient cohort data associated with a patient cohort sharing at least one characteristic with the patient represented by the patient model the system may determine a pathway in the treatment phase which the patient underwent based on a weighted calculation. The system may determine, based on dates of patient entries which are used to determine the status of the nodes, and further healthcare data, by which clinical pathway the patient was treated during the treatment phase. This allows a determination of the patient status to be made for nodes and/or directed edges for which patient entries are not stored.

The system may additionally transmit data indicating the at least one node and the at least one directed edge for which an attribute value cannot be selected based on the identifier for receipt by the user device; and receive, from the user device, data indicating the status of the at least one node and the at least one directed edge for which an attribute value cannot be selected based on the identifier. This allows a user of the user device to manually enter information which is missing from a patient model to identify the clinical pathway a patient has taken during treatment.

In an eleventh embodiment, there is provided a method of transmitting healthcare data to a user device, the user device being configured for use in analysing medical information, the method comprising:
maintaining, in a database, data representing a plurality of directed graphs, each directed graph representing at least part of at least one medical guideline and comprising a plurality of nodes and a set of directed edges, each node of the plurality of nodes being connected to at least one further node of the plurality of nodes by one of the set of directed edges, each directed graph comprising a primary node and terminating in at least one end node: maintaining, in a database, a plurality of patient models each comprising healthcare data associated with a respective patient;
receiving, from the user device, healthcare data comprising data identifying a patient and a medical condition;
selecting, based at least one the received data identifying the medical condition, a directed graph from the plurality of directed graphs;
selecting, based on the received data identifying the patient, a patient model from the plurality of patient models;
identifying, based on the selected patient model and the received healthcare data, a status of at least one of the nodes and at least one directed edge of the selected directed graph; and
transmitting data associated with the status of the at least one of the nodes and the status of the at least one directed edge for receipt by the user device.

In a twelfth embodiment, there is provided a computer program comprising a set of instructions, which, when executed by a computerised device, cause the computerised device to perform a method of transmitting healthcare data to a user device, the user device being configured for use in analysing medical information, the method comprising, at the computerised device:
maintaining, in a database, data representing a plurality of directed graphs, each directed graph representing at least part of at least one medical guideline and comprising a plurality of nodes and a set of directed edges, each node of the plurality of nodes being connected to at least one further node of the plurality of nodes by one of the set of directed edges, each directed graph comprising a primary node and terminating in at least one end node;
maintaining, in a database, data representing a plurality of patient models each comprising healthcare data associated with a respective patient;
receiving, from the user device, healthcare data comprising data identifying a patient and a medical condition;
selecting, based at least on the received data identifying the medical condition, a directed graph from the plurality of directed graphs;
selecting, based on the received data identifying the patient, a patient model from the plurality of patient models;
identifying, based on the selected patient model and the received healthcare data, a status of at least one of the nodes and at least one directed edge of the selected directed graph; and
transmitting data associated with the status of the at least one of the nodes and the status of the at least one directed edge for receipt by the user device.

### Brief Description of the Drawings

- Figure 1: shows a schematic block diagram of an example system in accordance with embodiments;
- Figure 1a: shows a schematic block diagram of an example system connected to a network in accordance with embodiments;
- Figure 2: shows an example of a directed graph in accordance with embodiments;
- Figure 3: shows a schematic block diagram of an event model in accordance with embodiments;
- Figure 4: shows a schematic block diagram of a patient model in accordance with embodiments;
- Figure 5: shows a flow diagram depicting an example of processing data in accordance with embodiments;
- Figure 6: shows a flow diagram depicting an example of processing data in accordance with embodiments;
- Figure 7: shows a flow diagram depicting an example of processing data in accordance with embodiments;
- Figure 8: shows a flow diagram depicting an example of processing data in accordance with embodiments;
- Figure 9a: shows an example of a display of a directed graph in accordance with embodiments;
- Figure 9b: shows an example of a display of a directed graph with the status of at least one directed edge indicated in accordance with embodiments;
- Figure 10: shows an example of a display of a user device in accordance with embodiments;
- Figure 11: shows an example of a display of a user device in accordance with embodiments;
- Figure 12: shows an example of a display of a user device in accordance with embodiments;
- Figure 13: shows an example of a display of a user device in accordance with embodiments;
- Figure 14: shows an example of a display of a user device in accordance with embodiments;

### Detailed Description

Embodiments will now be described in the context of systems, methods, and computer programs for providing information to a user of a user device, the device being configured for use in analysing medical information. Reference will be made to the accompanying drawings. In the following description, for the purpose of explanation, numerous specific details of certain examples are set forth. Reference in the specification to "an example" or similar language means that a particular feature, structure, or characteristic described in connection with the example is included in at least that one example, but not necessarily in other examples. It should further be noted that certain examples are described schematically with certain features omitted and/or necessarily simplified for ease of explanation and understanding of the concepts underlying the examples.

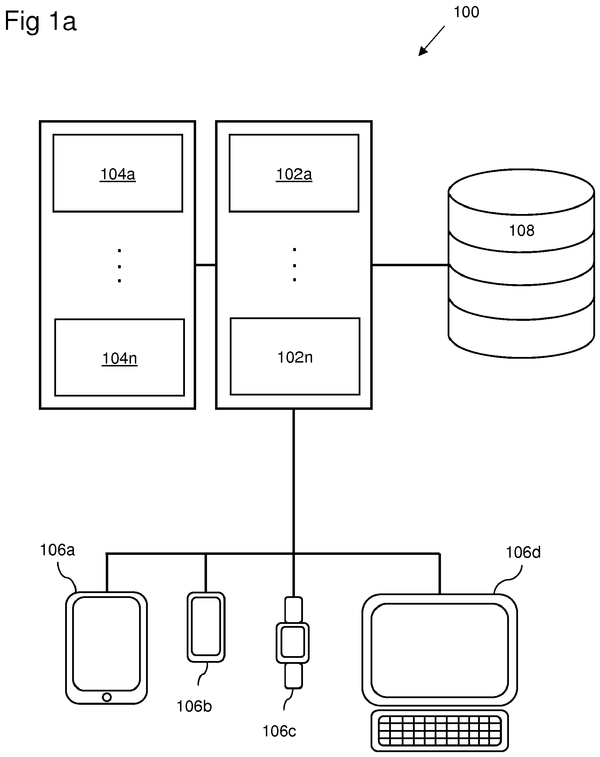

Figure 1a shows a diagram of a system 100 according to examples. The term "system" may refer to any combination of hardware, computer program code, functions, and virtualized resources embodied in a single, or across a plurality of devices. For example, the system 100 may comprise a single device housed in one location, for example, a computer in a hospital, or the system 100 may comprise a plurality of devices housed in the one location, connected over a local area network, for example, a mainframe computer communicatively coupled to at least one other computing device in a hospital. Systems comprising multiple computing devices may be more secure than systems comprising single computing device as multiple devices must fail for the system to become dysfunctional. It may be more efficient to use a system comprising a plurality of connected computing devices stored remotely from one another rather than having multiple systems.

In other examples, system 100 may comprise a plurality of remote devices. The plurality of remote devices may be connected over a metropolitan area network, a campus area network, or a wide area network, for example, the internet. The system 100 may comprise a plurality of servers and/or mainframe computing devices distributed in hospitals within a country. In other examples, the system 100 may be distributed across multiple countries.

The example system 100 shown in Figure 1a comprises at least one processor 102a-n. The at least one processor 102a-n may be a standard central or graphical processing unit (CPU or GPU), or a custom processing unit designed for the purposes described herein. Each of the at least one processors 102a-n may comprise a single processing core, or multiple cores, for example four cores or eight cores. In examples, wherein the system 100 comprises a plurality of processors 102a-n, the processors 102a-n may be embodied in a single device. In other examples the at least one processor 102a-n may comprise multiple processors remotely distributed within the system 100.

The example system 100 shown in Figure 1a comprises at least one memory 104a-n. The at least one memory 104a-n may be a non-transitory computer-readable memory, for example a hard-drive, a CD-ROM disc, a USB-drive, a solid-state drive or any other form of magnetic storage device, optical storage device, or flash memory device. The at least one memory 104an may be referred to as a storage medium or a non-transitory computer readable storage medium. The at least one memory 104a-n may be maintained locally to the rest of the system 100 or may be accessed remotely, for example, over the internet. The at least one memory 104a-n may store computer program code suitable for the function described herein. The computer program code may be distributed over multiple memories or may be stored on a single memory.

In an example, the system 100 refers to a system operable to provide healthcare data to a user device. The system 100 may be operated via a user device, by a user analysing medical information. For example, a doctor, a nurse, a clinical assistant, and others analysing medical information for a patient. Medical information may relate to data, for example, numerical test results from a diagnostic test. Medical information may also relate to qualitative diagnoses and notes relating to a patient or patients.

Figure 1a shows four examples of user devices 106a-d. A user device may be any combination of hardware and computer program code operable by a user and suitable for the function described herein. The user device 106a is a tablet computer, user device 106b is a smart phone, user device 106c is a smart watch, and user device 106d is a personal computer, for example a desktop or laptop PC, although other examples of user devices may be possible. User devices 106a-d may comprise any number of volatile or non-volatile memory, processors, and other electronic components. In some examples a user device 106a-d comprises multiple components distributed over a network. A user device may comprise any number of outputs, for example a display, a speaker, a tactile feedback system, an LED indicator, a transmitter or any other output. A user device 106a-d may comprise any number of inputs, for example, a microphone, a button, a camera, a receiver, or any number of sensors etc. In some examples the input and output of the user device 106a-d may be considered a user interface, for example, a touch screen or a combination of a screen and a keyboard. A user device 106a-d may be considered part of the system 100 or may not be part of the system 100 but may communicate with the system 100. In some examples a user device 106a-a is local to the system 100 and may be connected to the system 100 over a local area network, for example, a personal computer 106d in a hospital connected to a mainframe computer in the same hospital comprising the system 100. In other examples the user device 106a-d may connect to the system 100 via a wide area network.

The user device 106a-d may be configured for use in analysing medical information. For example, the user device 106a-d may comprise an application for presenting medical information to a user for analysis. In some examples there are multiple user devices 106a-d. The multiple user devices 106a-d may be communicatively coupled to one another. At least one user device 106a-d may be used to control the system 100.

In some examples, a user device 106a-d may be a proprietary device configured to be used in or with the system 100. For example, the user device 106a-d may be a proprietary computing device comprising a combination of firmware, software, and custom applications for providing data and/or other information to a user. For example, the user device 106a-d may comprise applications for displaying data received by and/or transmitted to the system 100 in a predetermined way.

In other examples, the user device 106a-d and the system 100 are comprised in the same device, for example, a desktop computer at a hospital.

In other examples, a user device 106a-d may be a commercially available computing device comprising any number of applications operable to access data at, receive data from, or transmit data to the system 100. For example, the system 100 may maintain at least one web page, hosted on a remotely accessible server. The user device 106a-d may comprise a web browser operable to access the at least one webpage and thereby facilitate communication with the system 100 and/or display data stored by the system 100 on the use device 106a-d.

The system 100 shown in Figure 1a comprises a database 108 for storing data according to embodiments described herein. The database 108 may be any structured set of data held in a computing device. For example, the database 108 may be structured data stored in the at least one memory 104a-n. In other examples, the database 108 may be stored elsewhere in the system, for example on a separate computing device. The at least one processor 102a-n may be communicatively coupled with the database 108 such that the at least one processor 102a-n may maintain the database 108. Maintaining the database 108 may comprise sending data to, receiving data from, or reconfiguring data in the database 108. In examples where the database 108 is stored on physical memory associated with the system 100, the at least one processor 102a-n may be configured to read and/or write data to the physical memory to maintain the database 108. The database 108 may comprise a plurality of databases associated with one another. The database 108 may be embodied by any suitable data structure.

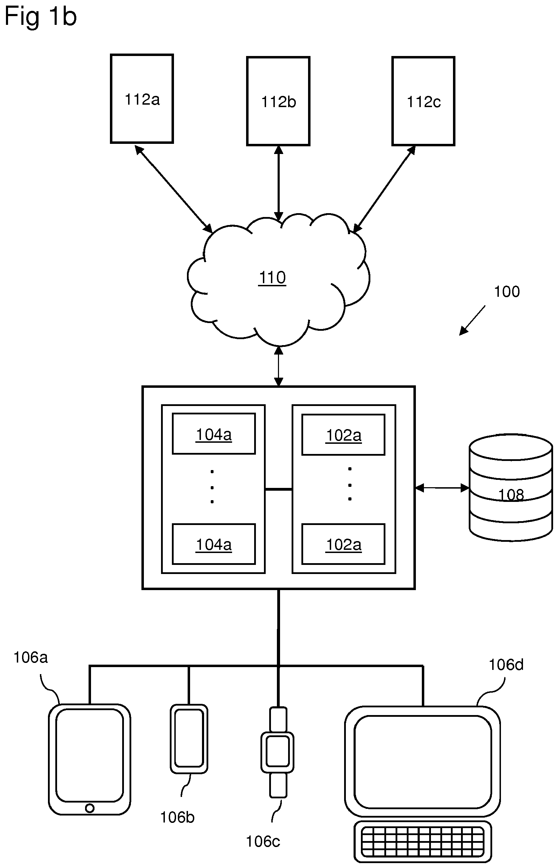

In some examples, the system may be able to communicate with other systems or remote data sources. In an example shown in Figure 1b, the system 100 is connected over a network 110 to at least one remote computing device 112a-c. For example, the system 100 may comprise a plurality of computers and servers located at a hospital, the system 100 may communicate with a computing system or device 112a at another hospital over the network 110 to send and/or receive medical data. The system 100 may simultaneously be in communication with a computing device 112b representing a medical guideline repository storing at least one medical guideline. A medical guideline repository may be embodied in any device storing at least one medical guideline. In some examples, a medical guideline repository may comprise a remotely accessible database storing at least one guideline, wherein the guideline is stored in a digital format and comprises metadata identifying the at least one guideline. For example, the medical guideline may be stored as a PDF and the metadata may comprise an indication of the name of the medical guideline, a date of publication, an indication of a disease to which the medial guideline relates, a country in which the medical guideline was first published or in which the medical guideline was designed to be applicable to, and any other which may be used to identify a guideline. More examples of identifying features of medical guidelines and their uses will be described later.

The system may also be in communication with a computing device 112c acting as a control device to control the operation of the system 100. For example, a remote computing device such as a personal computer or a server which may be used by an administrator to control the system 100.

Healthcare data as described herein may comprise data relating to: patient records (for example, results from diagnostic tests or therapeutic steps), medical guidelines (for example a directed graph as will be discussed below), statistical data, medical research, scientific articles, or any other medically or clinically relevant information. Healthcare data may be stored in any number of digital formats, the digital format in which the healthcare data is stored may be dependent on the type of healthcare data. For example, raw data relating to diagnostic results may be stored as plain text files, CSV files, or any other suitable file format. Some types of healthcare data may be stored in a human readable format or alternatively may be stored in computer interpretable language. In some examples, the system 100 may transmit healthcare data to a user device 106a-d in a computer interpretable format and the user device 106a-d may process the data and present it to a user in a human readable format.

A medical guideline may define clinical pathways for treating patients with a medical condition. In some examples, a medical guideline may be divided into a series of treatment phases. Examples of treatment phases may include: staging, initial treatment, active surveillance, recurrent treatment, and others. Clinical pathways may be defined by a series of clinical steps, wherein the choice of which clinical step to perform at a given time is dependent on at least a result from at least one previous clinical step. Clinical steps may include observations, decisions, events, diagnostic tests or therapeutic treatments to be delivered to a patient with a medical condition. Medical guidelines may be printed or published online in a digital format such as PDF. Medical guidelines may contain evidence and/or consensus-based recommendations for medical treatment pathways. Medical guidelines may also contain explanations and/or justifications for the clinical pathway defined within the respective medical guideline.

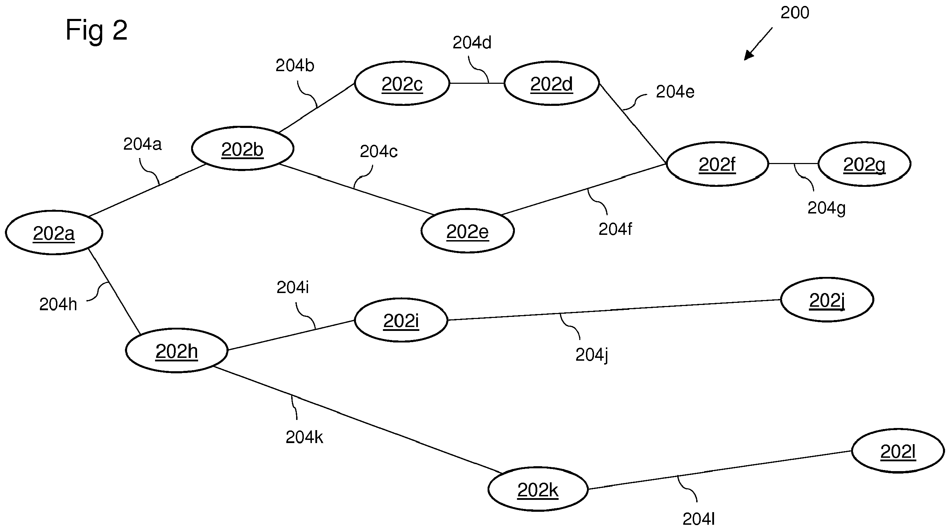

In examples described herein the system 100 may maintain in the database 108 a medical guideline represented by at least one directed graph. In some examples a set of directed graphs may be used to represent a medical guideline, for example with each directed graph representing a treatment phase within the medical guideline. In the forthcoming discussion reference may be made to a medical guideline being represented by a directed graph. However, it is acknowledged that a medical guideline may represented by a set of directed graphs and reference to a directed graph representing a medical guideline may refer to a directed graph representing at least part of a medical guideline. In an example, a set of directed graphs may connect to each other to form a medical guideline. The system 100 may be preinstalled with machine interpretable representations of the latest medical guidelines. An example of a directed graph can be seen in Figure 2. A directed graph 200 may be a graph comprising a plurality of nodes 202a-1 and a set of directed edges 204a-l, each node of the plurality of nodes 202a-1 connected to at least one other node of the plurality of nodes 202a-1 by one of the set of directed edges 204a-l. Each of the plurality of nodes 202a-1 of the directed graph 200 may represent a clinical step, for example, clinical steps described in the respective medical guideline represented by the directed graph 200. For example, the plurality of nodes 202a-1 may define a series of diagnostic tests and medical treatments which are recommended to be performed on a patient with a specific medical condition. The nodes 202a-1 may also define observational points and/or decisions that happen along a treatment pathway. The directed edges 204a-1 may define the direction and/or the order in which the clinical steps are to be performed when treating a patient with a particular medical condition. In some examples, the directed edges define conditions under which a patient is to move from undergoing a particular clinical step represented by a node, for example node 202c, to undergoing a different clinical step represented by a further node, for example 202d. Each of the set of directed edges may represent a conditional parameter value resulting from a said clinical step associated with one of the plurality of nodes connected thereto. In some examples, at least one of the set of directed edges may specify a user input to be received before traversing from one node to another node.

In an example, a directed graph may be maintained in JSON format, for example, as at least one list comprising unique identifiers representing nodes and directed edges of a directed graph. The entries in the at least one list may be linked to definitions or references in the respective guideline. In some examples this link may comprise other information, for example, a consensus-based weighting. The entries which represent directed edges may also comprise an association or link to nodes connected to the directed edge in the respective directed graph. The entries which represent directed edges may also comprise information on a direction from a first node connected to the directed edge to a second node connected to the directed edge. For example, the entries representing a directed edge 204c may comprise indicating that the directed 204c is connected to nodes 202b and 202e and that the direction along the directed edge 204c is from 202b to 202e. The directed graphs may comprise two layers of elements allowing modifications to be made at a clinic and/or clinical site without losing the information relating to the original directed graph.

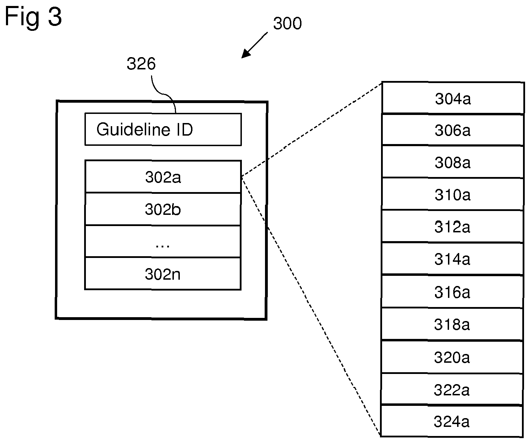

Further information relating to a directed graph may be maintained in an event model. Figure 3 illustrates an example of an event model for a directed graph. The event model 300 may comprise a list of entries 302a-n, representing events or steps, linked to respective nodes in the directed graph. The entries 302a-n may comprise any of: a unique identifier 304an, encoding in a medical coding system 306a-n, a label 308an, a type of step 301a-n(e.g. biopsy), a required patient attribute input 312a-n, a required patient attribute output 314a-n, annotations relating to: impact of event 316a-n, effectiveness of event 318a-n, cost of event 320a-n, duration of event 322a-n, invasiveness of event 324a-n, or any other relevant information. Thereby allowing important information contained in a medical guideline to be stored in an efficient manner allowing the use of medical guidelines as described herein. The event model 300 may comprise an identifier 326 relating the event model to a respective medical guideline. In Figure 3 only the data for the entry 302a is shown for clarity.

In some examples, a single directed graph may represent a medical guideline. In other examples a single directed graph may represent at least part of at least one medical guideline, for example a directed graph may represent a treatment phase within a medical guideline, and in still other examples, a directed graph may represent more than one medical guideline or at least part of more than one medical guideline.

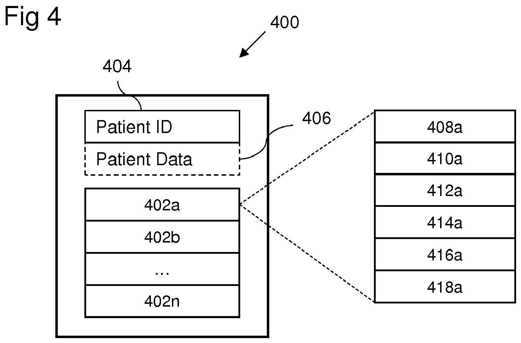

In certain examples described herein the system 100 may maintain, in the database 108, at least one patient model, the at least one patient model comprising healthcare data associated with a patient. For example, the patient model may comprise any of: data generated during clinical procedures such as diagnostic and/or therapeutic steps performed on a patient; general data relating to a patient such as age, gender, height; known conditions, risk factors, a patient identifier; or any other information classifying the patient. In some examples, the at least one patient model may be stored as a list comprising a plurality of patient entries. Each patient entry may comprise data relating to a patient attribute. Figure 4 shows an example of a patient model 400 comprising a list of patient entries 402a-n, a patient identifier 404, and in some cases other patient data 406. The patient entries 402a-n may comprise any of: a unique identifier 408an, an encoded identifier 410a-n such as an identifier in a medical encoding system, a natural language label 412a-n, a type of clinical step 414a-n (e.g. biopsy, scan, a physical assessment, etc.) a measurement unit 416a-n, and a patient attribute value 418a-n (for example, the result from a test). The patient entries may comprise an association between the encoded identifier 410a-n and the patient attribute value 418a-n also called an attribute value. In the example shown in Figure 4 only the data in patient entry 402a is shown for clarity. The encoded identifier may identify the clinical step to which the patient attribute value of the respective patient entry relates, wherein each clinical step is associated with a respective encoded identifier.

In some examples, a plurality of patient models relating to patients may be stored and/or maintained in a central database 108 or computing device. The patient models may be accessible through the system 100 over a network connection. In other examples, patient models may be stored locally with the clinical centre, for example a doctor's surgery or a hospital, at which the patient has been treated in the past or is currently receiving treatment. Patient models stored at one hospital in the system 100 may be accessed at remote locations through the system 100, for example, over the network 110.

In some examples, the system 100 updates patient models by accessing remote computing devices 112a-c over the network 110. For example, the system 100 may access medical testing equipment storing data relating to a patient, over the network 110 to update a respective patient model. The system 100 may access servers or other computing devices in hospitals which store medical information relating to patients. In some examples, the system 200 may continuously and/or regularly collect data about patients from various hospital information systems. Data may be collected at predetermined intervals for example, every hour, every day, etc. The size of the interval may be dependent on the size of the system 100 or the clinical centres. In some examples, data collection may be triggered by other events and/or messages occurring in the system 100. The retrieval of the data about the patients may be based on existing standards, for example, HL7, DICOM, FHIR. In other examples the retrieval of the data about the patients may be performed by using proprietary information about information storages available in a hospital. In some examples the retrieval of patient data is performed by receiving or accessing data, from external sources, comprising encoded identifiers such as SNOMED CT, LOINC, or Siemens internal coding system. In other examples, the system 100 may use natural language processing techniques to extract information from electronically stored notes and files relating to a patient. In some examples, a combination of both techniques may be used. The patient model may be represented in the system 100 as a set of resources conformant with FHIR standard and stored in an FHIR server.

The system 100 may analyse medical data, which may also be called clinical or healthcare data, stored in a plurality of patient models to derive statistical data to be used as further healthcare data. The system 100 may infer statistics on the patient histories represented in the patient models, for example, relative frequencies of conducted diagnostic and/or therapeutic steps. The system 100 may utilise a statistical and/or machine learning tool to infer statistical correlations, such as typical patient cohorts and typical duration, impact on quality of life, cost, etc. per patient cohort. Patient cohorts may be split into guideline conform and guideline non-conform subsets to analyse the typical adherence to the guideline for particular patient cohorts and to identify hypotheses for medical field studies by detecting statistically significant deviations from the guidelines. This may be used to help determine new pathways which are not yet in medical guideline.

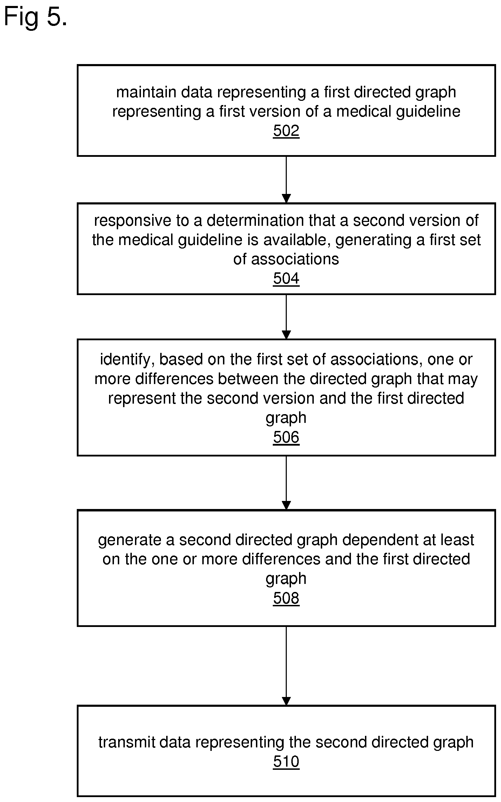

The following description of embodiments of the inventions will be described with reference to the example system of Figure 1. However, the following embodiments may be implemented in systems different to that of Figure 1. In an embodiment illustrated by a flow-chart in Figure 5, the system 100 may be operable to transmit healthcare data to a user device 106a-d, the user device 106a-d being configured for use in analysing medical information, the system 100 comprising at least one processor 102a-n and at least one memory 104a-n including computer program code configured to, with the at least one processor 102a-n, cause the system 100 to perform the step of maintaining, in a database 108, data representing a first directed graph representing at least part of a first version of a medical guideline, the first directed graph comprising a first plurality of nodes and a first set of directed edges, each node of the first plurality of nodes being connected to at least one further node of the first plurality of nodes by one of the first set of directed edges, the first directed graph comprising a primary node and terminating in at least one end node, as shown at block 502. In some examples, the first directed graph represents a first version of at least part of a medical guideline, for example, a single treatment phase, whereas in other examples the first directed graph may represent a whole first version of a medical guideline.

The program code may be configured to, with the at least one processor 102a-n, cause the system 100 to perform the step of, responsive to a determination that a second version of the medical guideline is available, generating a first set of associations, each being between one of a second, different, plurality of nodes of a directed graph that may represent at least part of the second version and a respective part of the second version, as shown in block 504.

In some examples, the system 100 may determine that a second version of the medical guideline is available based on a received command, for example from a control device such as remote computing device 112c or a user device 106a-d. In some examples, a received command may comprise an indication of a medical guideline for which a second version is available. The received command may include an indication of where the second medical guideline is stored. In other examples the received command may include the second version of the medical guideline. In some examples the second version is an updated version of the medical guideline.

In some examples, the system 100 may access a remote computing device 112a-c at predetermined intervals, for example, once a week, once a month, or any other suitable interval, to determine whether a second version of the medical guideline is available. For example, determining that a second version of the medical guideline is available may be performed by comparing metadata associated with the first medical guideline meta data associated with one or more further, different, medical guidelines stored at one or more medical guideline repositories 112b. For example, the system 100 may access at least one medical guideline repository 112b to check for a second version of the medical guideline associated with the first directed graph and comparing metadata associated with the first directed graph with metadata associated with metadata associated with at least one medical guideline stored at the at least one medical guideline repositories 112b. The metadata of each medical guideline may provide an indication of the publication date of the respective medical guideline, a version indicator, or any other information which may be used to compare and determine whether a medical guideline in the repository 112b is a second version of the medical guideline represented by the first directed graph.

In some examples, each of the first plurality of nodes may represent a clinical step. Each of the set of directed edges may represent a conditional parameter value resulting from a said clinical step associated with one of the first plurality of nodes connected thereto.

Generating the first set of associations may comprise retrieving the second version of the medical guideline wherein the second version of the medical guideline comprises passages which may be represented by a second, different, plurality of nodes of a directed graph. Retrieving the second version of the medical guideline may comprise downloading the medical guideline from a remote computing device 112a-c over a network 110. Alternatively, the medical guideline may be directly uploaded to the system 100 for example, via an external storage device such as a flash memory drive or a direct cable connection from an external computing device.

In some examples, the first set of associations may be between a plurality of unique identifiers and sections of the second version of the medical guidelines and/or to an event model representing the second version of the medical guideline. In some examples, the system 100 may generate the first set of associations when accessing and/or retrieving the second version of the medical guideline.

In some examples the program code may be configured to, with the at least one processor 102a-n, cause the system 100 to perform the step of identifying, based on the first set of associations, one or more differences between the directed graph that may represent at least part of the second version and the first directed graph, as shown at block 506. For example, the system 100 may detect a difference between a passage of the second version of the medical guideline and the respective passage in the original medical guideline, wherein the passage directly relates to at least one node. In some examples a difference may include an order of the nodes in a clinical pathway defined by the medical guideline. A difference may indicate a different clinical step to be performed at a stage in the clinical pathway. Differences may include additions to or omissions of information in the original medical guideline.

In some examples, identifying the differences comprises comparing data indicative of at least part of the first version of the medical guideline with data indicative of the second version of the medical guideline. For example, the whole text in the medical guideline represented by the first directed graph may be compared with the whole text in the second version of the medical guideline. Following the identification of differences in the text between the two versions, the system 100 may then determine if the detected differences relate to sections in the medical guidelines which are represented by nodes in the first plurality of nodes of the first directed graph, either by the first set of associations or otherwise. In other examples, only the respective parts or sections of the second medical guideline which comprise associations to a node of the second plurality of nodes are compared with the medial guideline represented by the first directed graph.

The program code may also be configured to, with the at least one processor 102a-n, cause the system 100 to perform the step of, generating a second directed graph dependent at least on the one or more differences and the first directed graph, as shown at block 508.

In some examples, generating the second directed graph may comprise determining associations between nodes and clinical steps described in the updated version of the medical guideline using natural language processing and modifying the respective nodes of the first directed graph based on these differences. Generating a second directed graph may comprise generating an event model representing events described in the medical guideline.

The program code may also be configured to, with the at least one processor 102a-n, cause the system 100 to perform the step of, transmitting data representing the second directed graph for receipt by the user device 106a-d, as shown at block 510. Transmitting data for receipt by the user device 106a-d may comprise sending data indicating the second directed graph over a network connection to the user device 106a-d. In some examples, the system 100 transmits data relating to the parts of the second directed graph which are different to the first directed graph.

In some examples, generating a second directed graph may comprise, based on a user input indicative of a decision in respect of at least one of the one or more differences, selectively using the at least part of first version or the second version to generate a respective part of the second directed graph. In a specific example this may comprise transmitting data representing at least one difference between the first directed graph and the directed graph that may represent at least part of the second version for receipt by the user device 106a-d and receiving, from the user device 106a-d, data indicating a decision to accept or reject at least one of the differences. For example, the system 100 may provide the identified changes to a user device 106a-d such that a user of the user device 106a-d may approve or decline any identified changes between the first directed graph and the directed graph that may represent at least part of the second version of the medical guideline. In some examples, it may not be possible to automatically generate a second directed graph, for example, where at least one of the first or second versions of the medical guideline is not structured appropriately. In such examples, the system 100 may transmit data indicative of at least one difference between the first and second versions of the medical guideline, for example differences in text comprised in the medical guidelines or differences in directed graphs representing at least part of medical guidelines. The user device may be used to review any differences and send a command to the system to accept or reject any differences.

In some examples, the first directed graph may comprise data indicative of at least one local modification made by a user of the system 100. The local modification made to the directed graph may be to reflect local or hospital specific policies. In some examples the local modification is specific to a user of the system 100. In some examples, a part of the second directed graph may be based on the at least one local modification. When generating the second directed graph 100 the system may copy the modifications made by the user of the system 100. In cases where the differences between the first directed graph and the directed graph that may represent at least part of the second version of the medical guideline comprise differences to parts of the first directed graph which comprise the at least one local modification, the system 100 may transmit data representing the differences for receipt by the user device 106a-d. The system 100 may then receive, from the user device 106a-d, data indicating a command either to (i) generate the second directed graph based on the second version of the medical guideline, (ii) generate the second directed graph based on the second version of the medical guideline and the at least one local modification, or (iii) generate the second directed graph based on at least part of the first version of the medical guideline. Thereby, local modifications to directed graphs made to reflect local policies may be maintained when updating a stored version of the medical guideline.

In other examples, the medical guideline repository may comprise at least one medical guideline comprising at least one modification made by a user of the system. In cases where the updated version of the medical guideline comprises at least one modification made by a user, data indicating the differences relating to these modifications made by the user may be transmitted to the user device 106a-d.

In some examples, the at least one processor 102a-n and at least one memory 104a-n including computer program code may be configured to, with the at least one processor 102a-n, cause the system to perform the steps of generating a second, different, set of associations, each being between one of a second, different, set of directed edges of the directed graph that may represent at least part of the second version and a respective part of the second version; identifying, based on the second set of associations, one or more further differences between the second set of directed edges and the first set of directed edges. This may be done similarly as described above in relation to the nodes. Differences between directed edges may include changes to conditional parameter values represented by the directed edges. Other differences may include differences in at least one node to which the directed edge is connected, the addition or omission of a directed edge, as well as other differences.

The program code may also be configured to, with the at least one processor 102a-n, cause the system 100 to perform the step of, generating the second directed graph dependent at least on the one or more further differences and the first directed graph. In an example, the second version of the medical guideline may comprise differences in parts relating to both nodes and direct edges and generating the second direct graph comprises generating a second directed graph comprising changes to both the directed edges and to the nodes.

In an example, the at least one processor 102a-n and at least one memory 104a-n including computer program code are configured to, with the at least one processor 102a-n, cause the system to perform the steps of maintaining, in a database 108, a patient model comprising healthcare data associated with a patient as described above; and determining, based on a combination of the first directed graph and the patient model, a first clinical pathway represented by at least some of the first plurality of nodes and at least one of the first set of directed edges. This may include comparing patient entries in the patient model with entries in the event model and the corresponding nodes and directed edges of the first directed. Examples of this are discussed further later in relation to other embodiments of the invention.

In the example wherein a first clinical pathway is determined, generating the second directed graph may comprise determining, based on a combination of the first clinical pathway and the one or more identified differences, a second clinical pathway represented by at least some of a third, different, plurality of nodes comprised in the second directed graph and at least one of a third, different, set of directed edges comprised in the second directed graph. If any of the identified differences comprise differences to nodes or directed edges in the first clinical pathway then determining the second clinical pathway may comprise determining a second clinical pathway up to but not including the respective nodes and/or directed edges comprising the one or more identifies differences.

In some examples, determining the second clinical pathway comprises, transmitting data indicative of a comparison between the first clinical pathway and the one or more differences, receiving, from the user device 106a-d, data indicating a command, and determining the second clinical pathway based at least on the received command. A user operating the user device 106a-d may review the differences between the nodes and directed edges in the first clinical pathway and the respective nodes and directed edges in the second directed graph. By operating the user device 106a-d, the user may send data indicating a command either to overwrite the first clinical pathway with a second clinical pathway, to erase the first clinical pathway, to attempt to merge the differences between the first clinical pathway and the second directed graph, or any other related function in order to generate a second clinical pathway comprising at least some of the third, different, plurality of nodes comprised in the second directed graph and at least one of the third, different, set of directed edges comprised in the second directed graph.

As discussed above, medical guidelines may be available for a variety of medical conditions. There may also be a plurality of medical guidelines relating to each medical condition. For example, different medical, governing, or regulatory bodies may publish and/or maintain medical guidelines specific to a particular region, country, practice group, hospital, etc. As also discussed above, a user of the system 100 may customise a guideline for specific use, for example, where a hospital does not have the facilities to perform clinical steps outlined in a medical guideline, an alternative diagnosis or treatment may be substituted. In some examples, medical guidelines may be modified for particular users. For example, a particular doctor or a group of doctors may be operating a clinical trial in which a clinical pathway not outlined in a general medical guideline may be used to treat a specific medical condition. In this case a specific medical guideline may be used for this doctor or group of doctors, further patients who are on the clinical trial may be associated with this specific medical guideline.

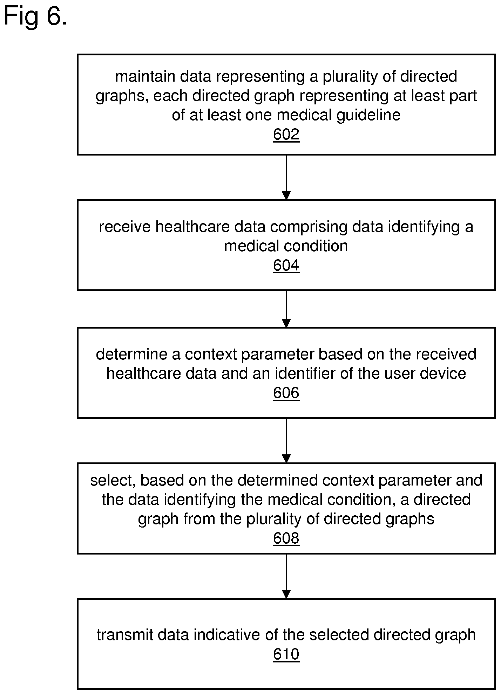

In an embodiment, illustrated by a flow-chart in Figure 6, the system 100 may be operable to transmit healthcare data to a user device 106a-d, the user device 106a-d being configured for use in analysing medical information, the system 100 comprising at least one processor 102a-n and at least one memory 104a-n including computer program code configured to, with the at least one processor 102a-n, cause the system 100 to perform the steps of maintaining, in a database 108, data representing a plurality of directed graphs, each directed graph representing at least one medical guideline and comprising a plurality of nodes and a set of directed edges, each node of the plurality of nodes being connected to at least one further node of the plurality of nodes by one of the set of directed edges, each directed graph comprising a primary node and terminating in at least one end node, as shown at block 602; receiving from the user device 106a-d, healthcare data comprising data identifying a medical condition, as shown at block 604. Receiving healthcare data identifying a medical condition from a user device 106a-d may comprise receiving from the user device 106a-d a specific indication of a medical condition such as a name of a medical condition, an identifier, or an indication of a clinical step which is specific to a particular medical condition, or any other healthcare data which may indicate a specific medical condition. In some examples the healthcare data received from the user device 106a-d comprises other healthcare data for example the results from a clinical step or data relating to a patient model.

The program code may also be configured to, with the at least one processor 102a-n, cause the system to perform the steps of determining a context parameter based on the received healthcare data and an identifier of the user device 106a-d, as shown in block 606; selecting, based on the determined context parameter and the data identifying the medical condition, a directed graph from the plurality of directed graphs, as shown in block 608. In an example, communications transmitted from the user device 106a-d may be encoded with an identifier of the user device 106a-d, such that the system 100 can identify from which user device 106a-d the communications are received. In other examples the identifier encoded in communications from a user device 106a-d may indicate a country or a hospital, or a particular user associated with the user device 106a-d. A context parameter may be any parameter which provides more selective criteria for determining which of the plurality of medical guidelines to select. The context parameter may comprise an indication of a location of the user device 106a-d. The context parameter may comprise an indication of a medical practitioner using the user device 106a-d.

As discussed above a medical guideline maybe specific to a hospital, a user, a country, as well as a date on which the guideline is accessed, a cohort to which the patient belongs, or even specific to a patient. As such, any parameter which further defines one of the stated, or any other suitable, criteria for selecting a medical guideline may be considered a context parameter. In some examples, a context parameter may comprise an indication from the user device 106a-d to not select a particular directed graph or to select a particular medical guideline.

The data indicative of the selected directed graph may be transmitted for receipt by the user device 106a-d as shown at block 610. This step may comprise, following the selection of a directed graph from the plurality of directed graphs, accessing the database in which data indicative of the selected directed graph may be stored to transmit data indicative of the selected directed graph to the user device 106a-d.

In some examples, the healthcare data comprising data identifying a medical condition is received via a network interface from the user device 106a-d over a wide area network.

Each of the nodes may represent a clinical step, for example, a clinical step described in a medical guideline represented by the selected directed graph. Each of the directed edges may represent a conditional parameter value resulting from a said clinical step associated with one of the nodes connected thereto.

The program code may also be configured to, with the at least one processor 102a-n, cause the system to perform the steps of maintaining, in a database 108, a plurality of patient models, each comprising healthcare data associated with a respective patient.

In some examples, the context parameter may comprise data indicative of one of the plurality of patient models. For example, the context parameter may comprise a unique identifier 404 identifying a patient. In other examples an indication of a patient model may be medical or clinical data identifying a patient model, for example, classifying data such as a height, gender, age, native language, name, assigned medical practitioner, etc. of a patient which may be comprised in patient data 406 of a patient model. The patient model may be associated with a directed graph from the plurality of directed graphs, for example, if the respective patient has been treated previously according to a directed graph. In this case determining a patient model from the plurality of patient models may also determine a directed graph from the plurality of directed graphs.

In some examples, the context parameter may comprise data associated with at least one patient entry. For example, data associated with a patient entry may be used to determine a patient model from the plurality of patient models where the data associated with the patient entry is unique. For example, a result from a clinical step, stored in a patient model may be used to identify a patient model. In some examples, a plurality of patient entries may be used to allow more accurate selection of the patient model.

In some examples more than one directed graph may be selected based on the context parameter and the identified medical condition. For example, for a particular medical condition, the system 100 may maintain a directed graph to be used for a specific patient cohort and a further directed graph to be used in a specific hospital. In the case that a patient falls within the specific patient cohort and is being treated at the specific hospital, data indicating both directed graphs may be transmitted to the user device 106a-d. For example, the at least one processor 102a-n and at least one memory 104a-n including computer program code may be configured to, with the at least one processor 102a-n, cause the system to perform the steps of: selecting, based on the context parameter and the data identifying the medical condition, a further directed graph from the plurality of directed graphs; and transmitting data indicative of the selected further directed graph for receipt by the user device 106a-d. In some examples, data indicative of the selected directed graph and data indicative of the selected further directed graph may be data indicating the names of the medical guidelines which the directed graphs represent. In other examples, data indicative of the selected directed graph and data indicative of the selected further directed graph may comprise sending data representing the nodes, the directed edges, the links between the nodes and directed edges, and in some examples, data relating to an associated event model of the directed graphs. The user device 106a-d may transmit data indicative of a command to choose one of the selected directed graphs and the selected further directed graph. After this choice, the system 100 may transmit data indicative of all the nodes and the directed edges of the chosen directed graph for display on the user device 106a-d.

In an example, the system 100 may, in response to receiving healthcare data select and transmit an excerpt from a medical guideline. The at least one processor 102a-n and at least one memory 104a-n including computer program code may be configured to, with the at least one processor 102a-n, cause the system to perform the steps of: maintaining, in a database 108, a plurality of textual excerpts from the at least one medical guideline;
selecting, based on the medical guideline represented by the selected directed graph, at least one textual excerpt from the plurality of textual excerpts; and
transmitting data indicative of the textual excerpt for receipt by the user device 106a-d. Transmitting data indicative of textual excerpts may comprise sending electronic files, for example, PDF files, .txt files, JPEGs, or any other suitable file format to transmit textual excerpts from a medical guideline. Thereby, a user of the user device may receive and review medical information relating to the medical guideline.

In some examples, the selected directed graph may comprise data indicative of a local modification of at least one of the nodes and/or at least one of the set of directed edges of the selected directed graph.

As discussed above, healthcare data may include data relating to patient records, for example, healthcare data may comprise analytics derived from data from a plurality of patients. The plurality of patient models may be grouped in terms of patient cohorts (i.e. a group of patients with at least one shared characteristic, for example, gender, age, height, ethnicity, known genetic conditions, etc.). The analytics may identify trends in the patient histories according to the patient models. For example, the propensity of a patient cohort to deviate from a particular medical guideline may be determined. In other examples, a particular hospital may be identified as providing clinical treatment in a strictly guideline conform way or may be identified as regularly deviating from medical guidelines. In some examples, machine learning techniques may be used to generate analytics. These trends may be used to determine shortcomings in medical guidelines and/or clinical pathways, as well as identifying beneficial treatments or more optimal clinical treatment pathways. In some examples, the analytics derived from the patient models may be stored with associations to other parameters. For example, analytics identifying a trend in a particular patient cohort may be stored with an association to that particular patient cohort. In other examples, analytics identifying trends in a particular hospital may be stored with an association to said particular hospital. The examples given of trends and groups in which trends may be determined are non-limiting and other ways of grouping patient data are possible. Other associations between analytics and other parameters are possible.

Healthcare data may include data relating to medical research. For example, healthcare data may comprise results, such as statistical results and analysis, from a medical study, such as a clinical trial. This may be directly loaded to the system 100 and stored in memory, for example in 104an. In other examples, the system 100 may access external computing devices 112a-c over a network 110 to retrieve results from a medical study. In some examples, results from a medical study may also include text, for example, a title, an abstract, a conclusion, a sample of the main body of text, or any other textual information derived from the medical study.

In some examples, medical studies may be mapped to nodes and/or medical guidelines by natural language processing techniques. For example, semantic relevance distances between a medical study and a medical guideline may be determined based on a natural language label in an event model associated with the medical guideline. The models *word2vec* and *doc2vec* may be used to determine semantic relevance distances. These models may first be trained on medical corpora to enable them to determine semantic relevance distances.

Healthcare data may comprise scientific articles. For example, textual extracts or a full text from a scientific article. In some examples, Figures from scientific articles are extracted and stored as healthcare data. Scientific articles may be retrieved and/or accessed by the system 100 over the Internet. Scientific articles may be directly loaded to the system 100 to be stored in memory. Scientific articles and/or extracts from scientific articles may be mapped to relevant medical guidelines and/or directed graphs based on semantic distances. These may be determined using cluster analysis which may utilise semantic distances provided by *word2vec* and *doc2vec* models trained on medical corpora, semantic distances based on references and citations, and semantic distances on the semantic subject and narrative mode of the documents. In some examples, the medical corpora are mapped to knowledge graphs using NLP techniques based on predefined semantic fields provided in the guideline event models.

Healthcare data may comprise other clinically relevant information, for example, as discussed above a user of the system may make modifications to stored directed graphs and/or other information relating to medical guidelines such as event models and textual extracts, data indicative of these modifications may be stored as healthcare data.

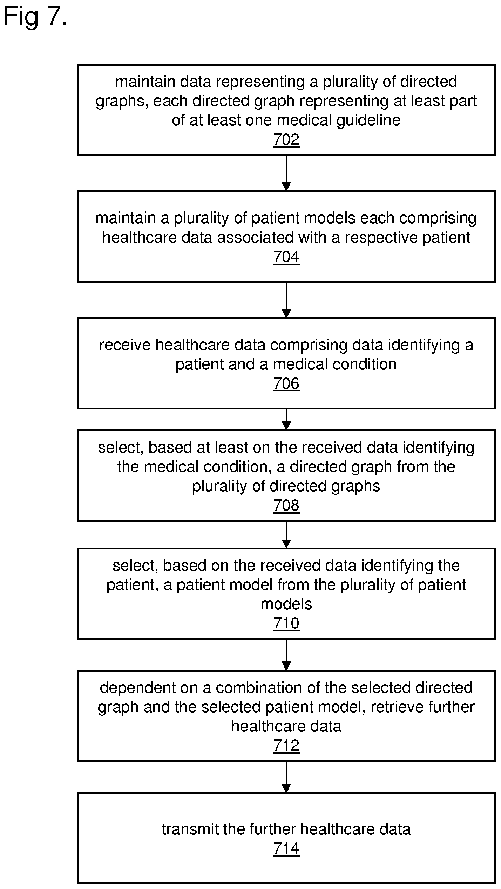

In an embodiment, illustrated by a flow-chart in Figure 7, the system 100 may be operable to transmit healthcare data to a user device 106a-d, the user device 106a-d being configured for use in analysing medical information, the system comprising at least one processor 102a-n and at least one memory 104a-n including computer program code configured to, with the at least one processor 102a-n, cause the system to maintain, in a database 108, data representing a plurality of directed graphs, each directed graph representing at least part of at least one medical guideline and comprising a plurality of nodes and a set of directed edges, each node of the plurality of nodes being connected to at least one further node of the plurality of nodes by one of the set of directed edges, each directed graph comprising a primary node and terminating in at least one end node, as shown at block 702, and to maintain, in a database 108, a plurality of patient models each comprising healthcare data associated with a respective patient, as shown at block 704. The system 100 may receive, from the user device 106a-d, healthcare data comprising data identifying a patient and a medical condition, shown at block 706. Subsequently the system 100 may select, based at least on the received data identifying the medical condition, a directed graph from the plurality of directed graphs, as shown at block 708. As discussed previously in relation to other embodiments, the system 100 may identify at least one context parameter and may select a directed graph from the plurality of directed graphs based on data identifying the medical condition and the at least one context parameter.

The system 100 may select a patient model from the plurality of patient models based on the received data identifying the patient model, as shown at block 710. Data identifying the patient model may comprise any data which may identify a patient, for example, a name, an identifier, patient entries and/or patient attribute values, etc.

The system 100 may, dependent on a combination of the selected directed graph and the selected patient model, retrieve further healthcare data, as shown at block 712. The system 100 may then transmit the further healthcare data for receipt by the user device 106a-d as shown at block 714. Thereby the user device 106a-d may receive further healthcare data to be used in analysing medical information. Further healthcare data may comprise at least one of a textual excerpt from a medical guideline associated with the selected directed graph, statistical information relating to a patient cohort associated with the selected patient model, a medical research paper, data resulting from a clinical trial, notes entered onto the system 100 by a medical practitioner, further medical data associated with the patient, a directed graph representing a different medical guideline, consensus based supplementary medical data, or any other medically relevant data. For example, the statistical information may be analytics identifying trends in a patient cohort, wherein the patient cohort may share at least one characteristic with the patient represented by the patient model.

Each of the nodes of may represent a clinical step and each of the directed edges may represent a conditional parameter value resulting from a said clinical step associated with one of the nodes connected thereto.

Further healthcare data may be organised into two categories: guideline conform, and additional enrichment. Guideline conform data may represent healthcare data which is derived directly from a medical guideline, for example, a directed graph, textual excerpts, etc. Additional enrichment data may represent further healthcare data which has not passed into a medical guideline, but which may be useful for a user. For example, scientific articles, clinical trial data, etc.

In an example where the retrieved further healthcare data comprises a directed graph representing a different medical guideline to the medical guideline represented by the selected graph, providing a different directed graph to the user device may allow a user to make a comparison between possible patient pathways and to deduce potential ways to increase the quality of the patient's treatment, which can be reflected in local hospital policies.

In some examples, the system 100 may maintain, in a database 108, at least one association between further healthcare data and at least one directed graph. The further healthcare data being retrieved according to the at least one association. Thereby the system 100 may retrieve further healthcare data that is relevant to the selected directed graph, to be transmitted to the use device. This allows the user device 106a-d to receive contextually relevant further healthcare data such that a user of the user device 106a-d, for example, a doctor analysing medical information associated with a patient, may be provided with supplementary medical information which may aid in making decisions relating to the treatment of the patient. This may allow doctors or other medical practitioners to consider more medical and clinical information when providing treatment to a patient. When operating the user device 106a-d in analysing medical information it may be desired to transmit relevant further healthcare data. For example, when analysing medical information relating to a patient with a medical condition about which little is known, it may be useful to retrieve a scientific research paper relating to said medical condition as the medical guidelines may have little information to assist in the prognosis.

In some examples, the system 100 may retrieve and pre-process further healthcare data. For example, the system 100 may retrieve medical corpus of scientific research information, from publication repositories, medical information services, etc., and may extract information relevant, based on semantic relevance, to a particular disease addressed in a medical guideline, the extracted information may be associated with the medical guideline and used as a reference to support decisions along the clinical pathway.

In some examples, the at least one association may be between the further healthcare data and a said node, and the further healthcare data may be retrieved according to the at least one association. This may allow a user of the user device 106a-d to be provided with further healthcare data relevant to a specific clinical step.

For example, a doctor analysing medical information relating to a patient may be directed to perform a type of biopsy on said patient, by the selected medical guideline. However, a research paper, associated with a node representing the biopsy may include a study that suggests that this type of biopsy may be ineffective for a patient belonging to a particular patient cohort. The patient to which the medical information the doctor is analysing related may be in this patient cohort. By transmitting the further healthcare data to the user device 106a-d, the doctor may determine that this type of biopsy should not be performed on the patient. Thereby the system 100 may allow an increase in efficiency, effectiveness of treatment, and a reduction in costs compared to other systems. In another example, the system 100 may, when a medical practitioner is analysing medical information relating to a selected patient model and a clinical step relating to a node on a selected directed graph, transmit to the user device 106a-d, data representing analytics relating to the clinical step and a patient cohort associated with the selected patient model. This may allow a medical practitioner to analyse medical information relating to a selected patient model and compare the selected patient model with similar patients.

In some examples, retrieving the further healthcare data is dependent on a status of at least one of the plurality nodes and/or at least one of the set of directed edges of the selected directed graph. This may allow more specific further healthcare data to be transmitted to the user device 106a-d.

The status of a node, in relation to a patient model, may be an indication as to whether the selected patient model comprises data relating to the node. For example, the status of a said node may be dependent upon availability of data associated with a clinical step represented by the node. For example, after determining that the selected patient model does not comprise data relating to a clinical step represented by the at least one node, it may be determined that the patient, represented by the selected patient model, has not undergone that clinical step and therefore the system 100 may not retrieve medical information relating to said clinical step. In other examples, after determining that the patient represented by the selected patient model has not undergone the clinical step represented by the at least one node, the system 100 may retrieve further healthcare data relating to the clinical step represented by the at least one node. For example, the system 100 may retrieve data indicating the effectiveness and/or the cost of a clinical step which the patient has not undergone. This may aid a medical practitioner in planning future care and/or treatment to be provided to a patient.

The status of a said directed edge may be dependent upon a combination of data associated with a clinical step represented by a said node connected thereto and a conditional parameter value represented by the directed edge. For example, the said directed edge may specify a minimum numerical value resultant from a clinical step, such as a diagnostic test, represented by a node connected to the said directed edge. The patient model may comprise data relating to the clinical step but the results from the test, which may also be considered attribute values, may not meet the minimum numerical value defined by the said directed edge, in this case the status of the said directed edge is an indication that the patient model comprises data that does not satisfy the conditional parameter value. In some examples the status may be an indication that the patient model: (i) comprises information that satisfies the conditional parameter value, (ii) comprises information that does not satisfy the conditional parameter value, or (iii) does not comprise information that either satisfies the conditional parameter value or does not satisfy the conditional parameter value.
In some examples the selected patient model may comprise a plurality of patient entries and determining a status of a said node and a said directed edge connected thereto comprises the steps of: maintaining a first association between at least one of the patient entries and an identifier from a plurality of identifiers; maintaining a second association between at least one of the nodes and an identifier from the plurality of identifiers; selecting, based on the first and second associations, a said attribute value associated with the node; and determining, based on a comparison of the attribute value associated with the node to a conditional parameter value represented by the directed edge, whether the conditional parameter value represented by the directed edge is satisfied.

The identifiers and the attribute values may each relate to a respective clinical step. For example, each patient entry may comprise an identifier in a coding system such as SNOMED CT, LOINC, Siemens internal coding system, or other coding systems. The attribute values may comprise the results from the respective clinical step. For example, wherein the clinical step comprises observation for changes in a patient condition, the attribute value may specify either a change, no change, or a minor change during the observation. The attribute value may comprise numerical data, for example results derived from a medical test such as a blood test. Each of the nodes of the selected directed graph may be associated with an identifier from the plurality of identifiers. In other examples, the nodes comprise links and/or associations to at least one event model which may comprise identifiers and/or other information relating to the clinical step represented by the respective node. In some examples the conditional parameter value may be a Boolean operator and determining that the Boolean operator is satisfied is by a direct comparison of the patient attribute value and the conditional parameter value. In some examples, natural language processing may be used to determine if data stored in the patient attribute value satisfies the Boolean operator.

In some examples, retrieving further healthcare data is dependent on a determination that a further, different, said directed graph based on the selected patient model does not correspond with the selected directed graph, for example, a the further, different, directed graph may deviate from the selected directed graph. A deviation may comprise a patient attribute value of the selected patient model not satisfying any directed edge in the selected directed graph. In other examples, a deviation may comprise the selected patient model comprising patient entries which comprise identifiers not matching any identifiers associated with the nodes of the selected directed graph. In this way, the system 100 may be able to provide supplementary healthcare data and/or information to a user of the user device 106a-d when the user is analysing medical information for a patient which has produced anomalous results and/or has been provided medical care which does not conform to the medical guideline represented by the selected directed graph. This may allow a medical practitioner to treat the patient more effectively.

In some examples, retrieving further healthcare data is dependent on a determination that the status of at least one of the nodes and/or at least one of the set of directed edges of the selected directed graph cannot be determined based on the selected patient model. For example, the patient model may be missing data, such as attribute values like life expectancy, relating to nodes in the selected directed graph. The retrieval of further healthcare data may comprise transmitting an indication of the missing attribute value to the user device 106a-d and may receive, from the user device 106a-d, a patient attribute value corresponding to the missing entry.

In other examples, upon determining that the status of at least one node and or at least one directed edge cannot be determined based on the plurality of patient attribute values the system 100 may retrieve a textual excerpt from a said medical guideline represented by the selected directed graph, the textual excerpt providing information relating to the clinical step represented by the at least one node and/or a conditional parameter value represented by the at least one directed edge.

In some examples, the system 100 may store and update ratings for the relevance of further healthcare data being transmitted to the user device 106a-d. For example, the at least one processor 102a-n and at least one memory 104a-n including computer program code may be configured to cause the system 100 to maintain, in a database 108, an association between a plurality of state parameters and the further healthcare data, the state parameters being for use in processing a decision at a node of a directed graph. For each item of further healthcare data, for example a scientific paper, a textual excerpt from a guideline, a statistic, etc. the system 100 may maintain a plurality of state parameters, each state parameter may represent the relevance of the item of further healthcare data to a node and/or edge of a directed graph. In an example, a scientific paper relating to the operability of lung cancers may be associated with a state parameter θ which specifies the relevance of this scientific paper to a diagnostic step for determining the types of nodules of a lung cancer, in this case the state parameter may have a low value as the scientific paper is not relevant to the diagnosis of the types of nodules of a lung cancer. The scientific paper may also be associated with a state parameter β which specifies the relevance of this scientific paper to an operation involving lung cancer, in this case the state parameter β may have a high value as the paper is relevant to operations involving lung cancer. When retrieving further healthcare data dependent on a status of the node representing the diagnostic step for determining the type of nodules of the lung cancer the system 100 may not select the scientific paper relating to the operability of lung cancers as the state parameter θ indicates a low relevance. Whereas when retrieving further healthcare data dependent on a status of a node representing an operation relating to a lung cancer the system 100 may select and retrieve the scientific paper relating to the operability of lung cancers, as the state parameter β has a high value. Thereby appropriate and relevant further healthcare data may be retrieved and transmitted to the user device 106a-d.

In some examples, the system 100 may be caused to receive, from the user device 106a-d, data indicating a rating associated with the retrieved further healthcare data and may process the rating to modify a said state parameter based on the received rating. Thereby a user operating the user device 106a-d may update the state parameters which indicate the relevance of further healthcare data to a node and/or edge of a directed graph. This may allow dynamic updating of further medical information transmitted to the user device 106a-d. For example, as medical research papers age they may become less relevant and as such may be transmitted to the user device 106a-d less frequently. In other examples, statistical data which proves to be unreliable may be rated poorly and thereby may be transmitted to the user device 106a-d less frequently. The converse may also occur, in that particularly useful or relevant healthcare data may be transmitted to the user device 106a-d more often.

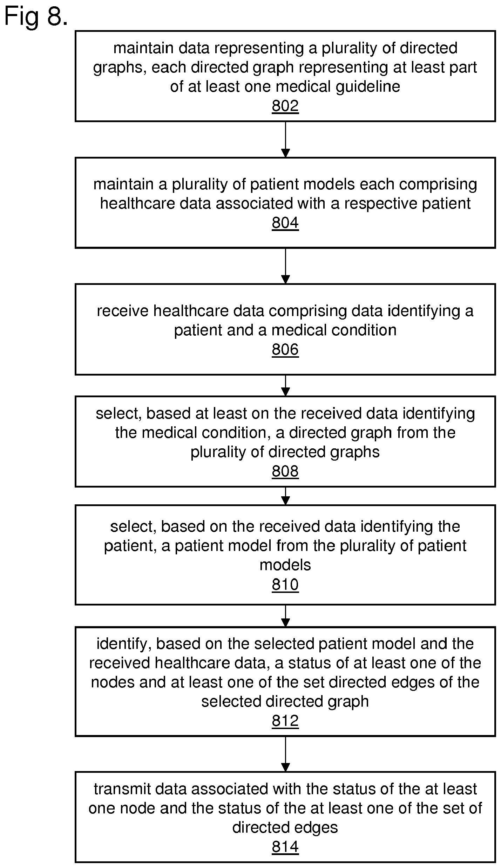

In an embodiment, illustrated by a flow chart in Figure 8, the system 100 may be operable to transmit healthcare data to a user device 106a-d, the user device 106a-d being configured for use in analysing medical information, the system comprising at least one processor 102a-n and at least one memory 104a-n including computer program code, the at least one memory 104a-n and computer program code configured to, with the at least one processor 102a-n, cause the system perform the steps of; maintaining, in a database 108, data representing a plurality of directed graphs, each directed graph representing at least part of at least one medical guideline and comprising a plurality of nodes and a set of directed edges, each node of the plurality of nodes being connected to at least one further node of the plurality of nodes by one of the set of directed edges, each directed graph comprising a primary node and terminating in at least one end node, as shown at block 802; maintaining, in a database 108, a plurality of patient models each comprising healthcare data associated with a respective patient, as shown at block 804; receiving, from the user device 106a-d, healthcare data comprising data identifying a patient and a medical condition, as shown at block 806; selecting, based at least on the received data identifying the medical condition, a directed graph from the plurality of directed graphs, as shown at block 808; selecting, based on the received data identifying the patient, a patient model from the plurality of patient models, as shown at block 810; identifying, based on the selected patient model and the received healthcare data, a status of at least one of the nodes and at least one of the set of directed edges of the selected directed graph, as shown at block 812; and transmitting data associated with the status of the at least one of the nodes and the status of the at least one of the set of directed edges for receipt by the user device 106a-d, as shown at block 814. Thereby a user, operating a user device 106a-d to analyse medical information relating to the patient represented by the selected patient model may be informed of the status of the patient. For example, the data transmitted to the user device 106a-d may indicate which clinical steps in the medical guideline represented by the selected directed graph that the patient has undergone, the results of these clinical steps, the patient's current status, and the potential future clinical steps which may be performed on the patient. As described in relation to other embodiments, each of the nodes may represent a clinical step, for example, a clinical step described in a medical guideline. Each of the directed edges may represent a conditional parameter value resulting from a said clinical step associated with one of the nodes connected thereto.

As discussed in relation to previous embodiments, the status of a said node may be dependent upon availability of data associated with a said clinical step represented by the node. The status of a said directed edge may be dependent upon a combination of data associated with a clinical step represented by a said node connected thereto and a conditional parameter value represented by the directed edge. The patient model may comprise a plurality of patient entries, and determining a status of a said node and a said directed edge connected thereto may comprise the steps of: maintaining a first association between at least one of the patient entries and an identifier from a plurality of identifiers; maintaining a second association between at least one of the nodes and an identifier from the plurality of identifiers; selecting, based on the first and second associations, a said attribute value associated with the node; and determining, based on a comparison of the attribute value associated with the node to the conditional parameter value represented by the directed edge, whether the conditional parameter value represented by the directed edge is satisfied.

In some examples, the at least one processor 102a-n and at least one memory 104a-n including computer program code are configured to, with the at least one processor 102a-n, cause the system to: determine, based on the identified status of the at least one of the nodes and the at least one directed edge of the selected directed graph, whether a further, different, said directed graph based on the selected patient model corresponds with the selected directed graph; and, dependent on the determination, to transmit data indicating the determination for receipt by the user device 106a-d. In some example, the data indicating the determination may comprise an indication of for which node(s) and/or directed edge(s) the selected patient model deviates from the selected directed graph. In other examples the data indicating the determination may comprise an indication of the patient entries in the selected patient model which deviate from the selected directed graph. Thereby a user operating the user device 106a-d may be informed that the patient represented by the selected patient model may be deviating from the medical guideline and/or may require further assessment or analysis.

In some examples, the at least one processor 102a-n and at least one memory 104a-n including computer program code are configured to, with the at least one processor 102a-n, cause the system to determine, based on the identified status of the at least one of the nodes and the at least one directed edge, a start date and an end date of at least one treatment phase associated with the at least one medical guideline and transmit data indicating the start and the end date of the at least one treatment phase for receipt by the user device 106a-d. As discussed above, a medical guideline may be divided into treatment phases. By determining the status of at least one node and/or directed edge of the selected directed graph, the system may determine that a patient represented by the selected patient model has started or finished a treatment phase. A treatment phase may be represented by at least some of the plurality of nodes connected by at least one of the set of directed edges. This information may be useful when determining whether a patient completed a treatment phase in a time interval set out in the medical guideline or whether the patient represented by the selected patient model completed a treatment phase in a shorter time or a longer time than prescribed in the medical guideline. Thereby allowing a user of the user device 106a-d to analyse how guideline conform a patient has been treated, or whether the medical guidelines may not be applicable to a particular case.

Having identified the at least one treatment phase, the system 100 may retrieve further healthcare data associated with the at least one treatment phase. For example, the system 100 may retrieve an extract from a medical guideline describing the treatment phase, or the system 100 may retrieve statistical data indicating trends in other patients in relation to the at least one treatment phase, for example, average time to complete the treatment phase.

The system 100 may then transmit the further healthcare data associated with the at least one treatment phase for receipt by the user device 106a-d.

In some examples, following the identification of the at least one treatment phase the system may identify, for the at least one treatment phase, at least one node and at least one directed edge for which an attribute value cannot be selected based on the identifier and may determine the status of the at least one node and the at least one directed edge using the selected patient model and further healthcare data. For example, the system 100 may determine the status of nodes and edges either side of the nodes and directed edges defining the at least one treatment phase, by comparing patient entries with nodes and directed edges in the selected directed graph. Within the set of nodes and directed edges defining the treatment phase there may be multiple routes of clinical steps the patient may have taken during treatment. The route the patient has taken between the nodes and directed edges for which a status has been determined, may be determined based on the patient data in the patient model and further healthcare data. For example, a patient cohort to which the patient belongs may be identified based on the selected patient model and further healthcare data may comprise an indication of the most common route taken by the respective patient cohort.

In some examples, the system 100 may transmit data indicating the at least one node and the at least one directed edge for which an attribute value cannot be selected based on the identifier for receipt by the user device 106a-d and may receive, from the user device 106a-d, data indicating the status of the at least one node and the at least one directed edge for which an attribute value cannot be selected based on the identifier. Thereby if the status of a node and/or a directed edge cannot be determined based on a combination of the selected patient model and the selected directed graph, the system may request a user to send the missing information to the system 100 such that the status may then be determined.

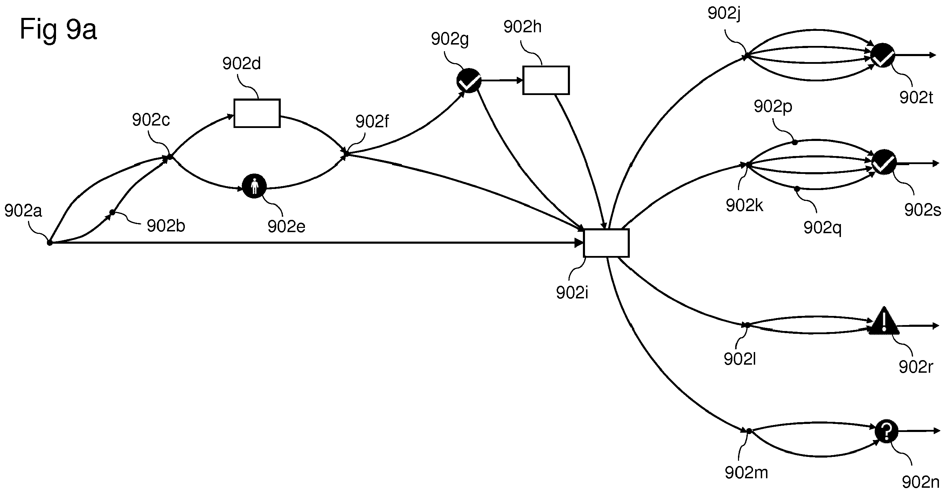

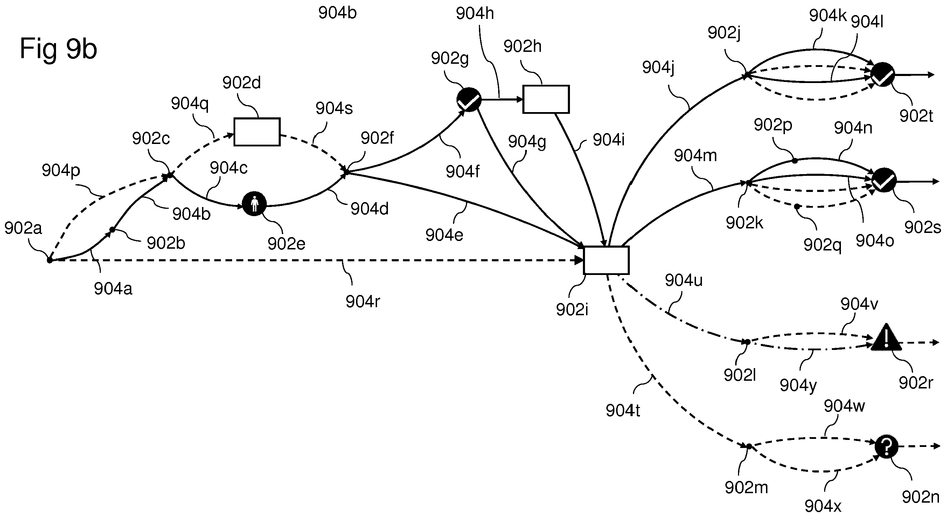

In some examples, transmitting data to the user device 106a-d may comprise transmitting data to cause the user device 106a-d to display the data. As described above the user device 106a-d may be a proprietary user device 106a-d running an application configured to present healthcare data to a user. In other examples, the user device 106a-d may run a web browsing application and access an application hosted by the system 100 over a wide area network. Examples relating to user interfaces on the user device 106a-d are set out in Figures 9-14 and will be described in relation to the above embodiments.

Figure 9 sets forth an example of a directed graph representing a medical guideline. The directed graph comprises a plurality of nodes representing clinical steps described in a guideline. The directed graph also comprises a plurality of directed edges, each directed edge being connected to at least one node of the directed graph. The directed edges may represent conditional parameter values which define conditions for moving from a first node connected to the directed edge, along the directed edge to a second node connected to the directed edge.

In some examples, transmitting data representing the directed graph comprises transmitting a list of identifiers of nodes and directed edges including associations between directed edges and nodes. In some examples, when displaying a directed graph, the user device 106a-d may use different icons to represent different nodes. For example, nodes which represent diagnostic steps may be displayed using a first icon and nodes representing therapeutic steps may be displayed using a second icon. In the example set forth in Figure 9a, at node 902e an icon showing a human figure may indicate a patient's current position on the clinical pathway. Icons comprising a check mark such as those used to represent nodes 902g, 902t, and 902s in Figure 9a may be used to indicate a recommended clinical step. Icons comprising question marks, for example the icon in Figure 9a representing node 902n, may indicate that the system 100 does not have enough data, for example, missing patient entries in the patient model, to determine whether the clinical steps represented by the nodes having question marks are recommended. Icons comprising caution signs, also known as hazard or warning signs, for example the icon in Figure 9a representing node 902r, may indicate that the system 100 does have all necessary data to determine whether the clinical steps represented by the nodes having caution signs are recommended but that these clinical steps are not recommended. For example the system 100 may have patient entries relating to all the directed edges up to the node 902r, including for example data relating to directed edges 904u, 904y, 904v but that the patient entries relating to these directed edges do not satisfy the conditions represented by these directed edges and therefore the clinical step represented by node 902r is not recommended. Some nodes may be represented by blocks, for example nodes 902d, 902h, and 902i of the Figure 9, which may display labels such as identifiers of the clinical steps or other text relating to the clinical steps.

Recommended clinical steps may be clinical steps for which the conditional parameter values of the directed edges leading from the patient's current position to the respective next option are satisfied by patient attribute values comprised in the patient model. Some options may not be recommended due to the conditional parameter values of the directed edges leading from the patient's current position to the respective next option not being satisfied by entries in the patient model. In examples in which one or more patient attribute values are missing from the patient model but are required to determine the next option in the clinical pathway, a user of the device may be alerted, for example, by transmitting an alert to the user device, before a tumour conference, whether all the patient attributes required for a medical guideline conform decision to be made are available.

In examples where the status of at least one node and/or the status of at least one directed edge is transmitted to the user device 106a-d, an indication of the status may be integrated into the display of the directed graph when displayed on the user device 106a-d. Figure 9b shows an example of the same directed graph as in Figure 9a but also with an indication of the status of at least one node and at least one directed edge integrated into the display. In the example shown in Figure 9b the directed edges 904a-c for which the conditional parameter values are satisfied by a selected patient model are shown in solid lines. Solid lines may also be used to indicate a recommended patient pathway, in this case indicated by directed edges 9d-o. Those directed edges for which the conditional parameter values are not satisfied by the selected patient model, may be shown in broken lines as shown by directed edges 9p-y. In other examples, colour, transparency, or other methods may be used to delineate between the status of directed edges. An indication of the status of nodes of the directed graph for which the status has been determined may also be integrated in a similar way as with the directed edges.

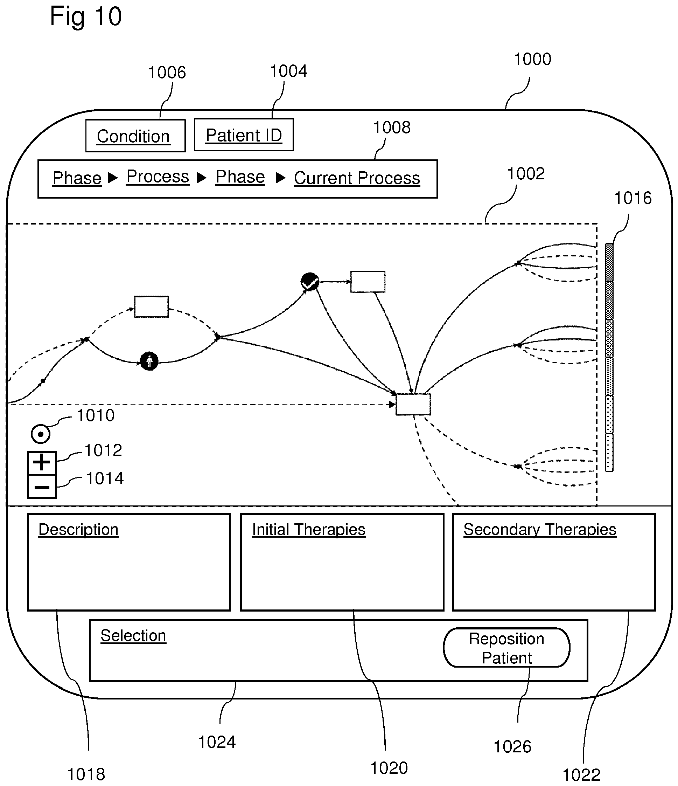

Figure 10 shows an illustrative example of a user interface of an example user device 106a-d. In the example set forth in Figure 10 the user device 106a-d may be in an overview mode in which the directed graph 1002 illustrates clinical pathways described in a respective medical guideline. The directed graph may comprise information relating to the status of at least one node and at least one directed edge, which may be integrated into the display by, for example, colour, transparency, size of elements in the directed graph, and or patterns or textures such as broken lines. The directed graph 1002 shown on the user interface may be generated based on a combination of data representing the selected directed graph and data associated with the status of at least one node and at least one directed edge.

As discussed above, the system 100 may determine whether a patient's clinical pathway deviates statistically from the clinical pathways of other patients in the patient's cohort. In this case, the display of the directed graph may indicate the standard and/or most common clinical pathway for the patient's cohort. The user interface may also indicate, for example display a warning, when the patient deviates from this clinical pathway.

The user interface may display a Patient ID field 1004 comprising information relating to a patient for which medical data is being analysed, for example, a name, a unique identifier, a date of birth, and/or any other identifying information relating to the patient.

The user interface may display an indication of the condition of the patient in a block 1006 displaying the condition which may comprise a name of the condition, an encoded identifier of the condition, or any other relevant information.

A user may be able to navigate and alter the view of the directed graph 1002 by the use of a touch screen or a cursor by clicking and dragging the directed graph. The user may centre the directed graph on a current patient position, which may be determined by the status of at least one node and at least one edge of the directed graph, by using the button 1010 to centre the display onto the portion of the directed graph to which the most current data in the patient model relates. The user may also be able to zoom in and out of a portion of the directed graph using the buttons 1012 and 1014.

The position of nodes on a directed graph may be indicative of information in the medical guideline represented by the directed graph. For example, the horizontal position of a node shown in the example of Figure 10 may represent the time at which the clinical steps have or are scheduled to take place. The vertical position of a node on the directed graph 1002 may be an indication of the invasiveness of the clinical step. A bar indicating a scale of invasiveness in relation to vertical position may be provided as the bar 1016, wherein nodes positioned higher with respect to the bar 1016 represent more invasive clinical steps and nodes positioned lower with respect to the bar 1016 represent less invasive clinical steps.

A user may select a node and/or a directed edge of the directed graph 1002 using a user interface, for example by touching a portion of a touchscreen displaying the node, using a cursor, using a voice command, or other methods of interfacing with the user device 106a-d. Upon selection of a node and/or directed edge, the system 100 may retrieve further healthcare data and may transmit said further healthcare data to the user device 106a-d. The further healthcare data may be displayed in blocks for example blocks 1018, 1020, and 1022 shown in Figure 10. Block 1018 may display a description of the clinical step represented by the node, for example, an extract from the respective medical guideline may be provisioned and displayed at this block 1018 or a description relating to the conditional parameters of the directed edge may be displayed. This further healthcare data may be retrieved according to semantic distances between the patient model and semantic description of the node and/or directed edge. In other examples the further healthcare data is retrieved using a specialized service to search scientific corpora, for example, using Science Direct®, or PubMed®. Block 1020 may give overview information about any initial therapies specific to the medical condition the medical guideline is associated with. Block 1022 may display information comprising explanations and/or a list of secondary therapies.

The system 100 and/or the user device 106a-d may automatically modify the size of the blocks 1018, 1020, and 1022 to display the healthcare data. In some examples the blocks 1018, 1020, and 1022 comprise scrolling interfaces such that in examples where the healthcare data cannot all be displayed at once a user can access a portion of the healthcare data by scrolling.

The user interface may comprise a block 1024 showing information relating to a selected node and a button 1026 may be provided to allow a user to reposition the patient to the selected node. For example, in a situation where a patient has undergone a clinical step but data relating to this has not been input to the system 100, a user of the user device 106a-d may reposition the patient along the directed graph 1002.

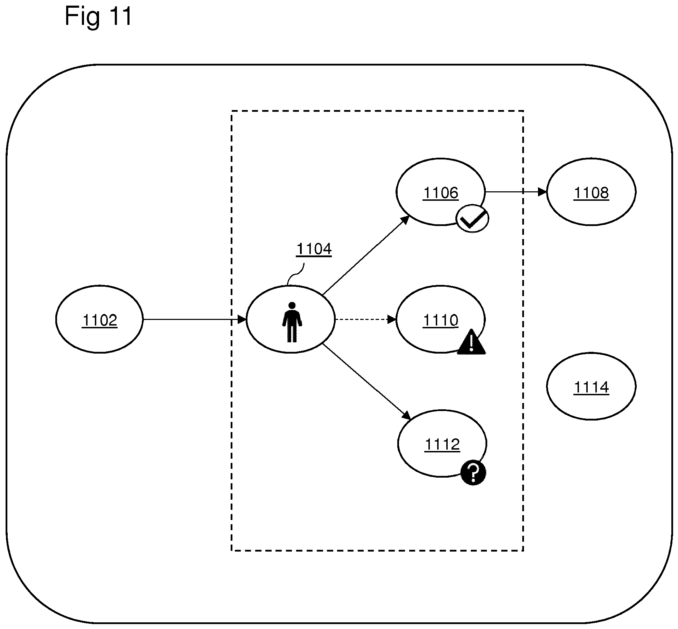

In an example set forth in Figure 11, the user device 106a-d may be used in a targeted guideline view in which a portion of a directed graph is displayed. The portion of the directed graph which is displayed may comprise an icon 1102 representing a most recent previous clinical step, an indication of what this clinical step was may be displayed alongside the icon. An icon 1104 may indicate a current position of a selected patient along the clinical pathway, in some examples this may include displaying information relating to the current clinical step. An icon 1106 may represent a recommended next clinical step, this may include an indication that this step is recommended. For example, the icon 1106 may be displayed in a particular colour, with a thicker border than other icons, or as in Figure 10 with a check mark. There may be displayed, an icon 1108 indicating subsequent clinical steps to the recommended clinical steps. Further icons may also be displayed such as icons 1110 and 1112 representing clinical steps which are not recommended and for which a recommendation cannot be determined respectively. In some examples, other tangential nodes may be represented by icons 1114 to provide an indication of other clinical pathways. This display mode may allow user to focus on a particular decision between clinical pathways for a patient.

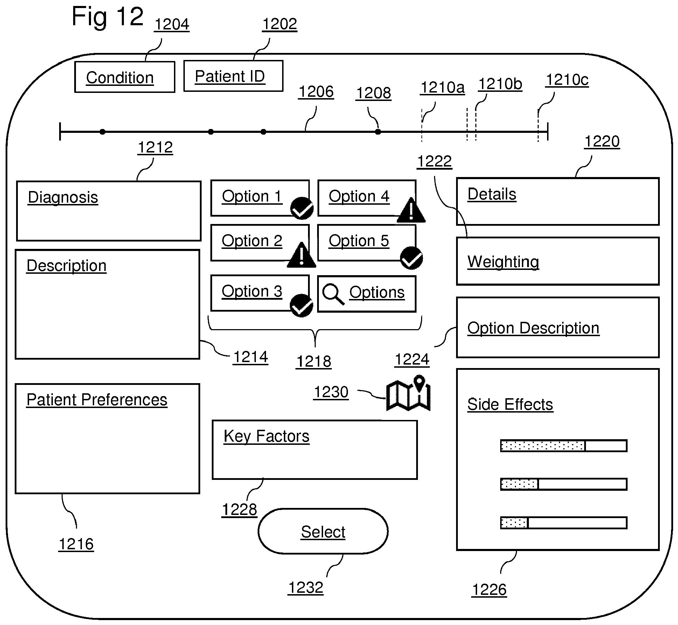

In an example set forth in Figure 12 the user interface may be in a decision options view in which a clinical step to be performed on a patient can be selected. The user interface may display a Patient ID field 1202 comprising information relating to a patient for which medical data is being analysed, for example, a name, a unique identifier, a date of birth, and/or any other identifying information relating to the patient.

The user interface may display an indication of the condition of the patient in a Condition field 1204 which may comprise a name of the condition, an encoded identifier of the condition, or any other relevant information.

The user interface may display a timeline 1206 summarising previous clinical steps in the patient history. For example, node 1208 may represent a previous clinical step such as a diagnostic scan. Lines 1210a-c may indicate important dates in the patient history. For example, 1210a may indicate the staging of the patient in a treatment phase, 1210b may indicate the current date and 1210c may indicate the projected end date of the current treatment phase.

Further healthcare data relating to the medical guideline being used and the patient model being used may be displayed. For example, data relating to the diagnosis of the patient may be displayed at a block 1212, a description of the medical condition such as an extract from a medical guideline may be displayed at block 1214, and patient preferences indicating considerations to be made when deciding which treatment a patient will receive may be displayed at block 1216.

The user device 106a-d may display a list of available options 1218 at this stage of the treatment. Icons representing these options may comprise indications as to whether they are recommended options (check mark), not recommended options (triangular warning symbol), or options for which a recommendation cannot be provided (question mark). In some examples an icon is available to search for further options.

Upon an interaction with an option, for example by clicking an option 1218 with a cursor, the system 100 may transmit further healthcare data relating to the selected option. For example, the system 100 may transmit to the user device 106a-d for display details 1220 relating to the selected option, a weighting of the option 1222 such as a weighting set out in a medical guideline, a description of the option 1224 including any information about the risks, costs, effectiveness etc., and side effects 1226 which may be displayed as a chart displaying the risk of any potential side effects. In an example, when a user clicks on an option which is not recommended e.g. option 2 or option 4 1218, the system 100 may transmit to the user device 106a-d data from the patient model which was used in making this determination. This data may be displayed in the details block 1220. Thereby, a user of the user device 106a-d may be presented with the reason why an option is not recommended and may dispute said decision accordingly.

Key factors which are used to determine which options are recommended and which options are not recommended may be displayed at block 1228. The key factors shown at block 1228 may be modifiable by the user to better reflect situations where the key factors may be subjective or dependent on contextual information. A button 1230 may be provided to select between a view of a directed graph and the decision options view, such that previous and potential future clinical steps may be analysed when making a decision about a treatment option.

One of the options 1218 may be selected using a button 1232 on the user interface. The blocks shown in Figure 12 are illustrative examples and sizes and positions of these may be different to those shown. In some examples the sizes of the blocks are dynamic. In other examples the blocks may be of constant size, but the information displayed within may be dynamic, for example, a scrolling function may be included to review text which does not fit within the allocated block size .

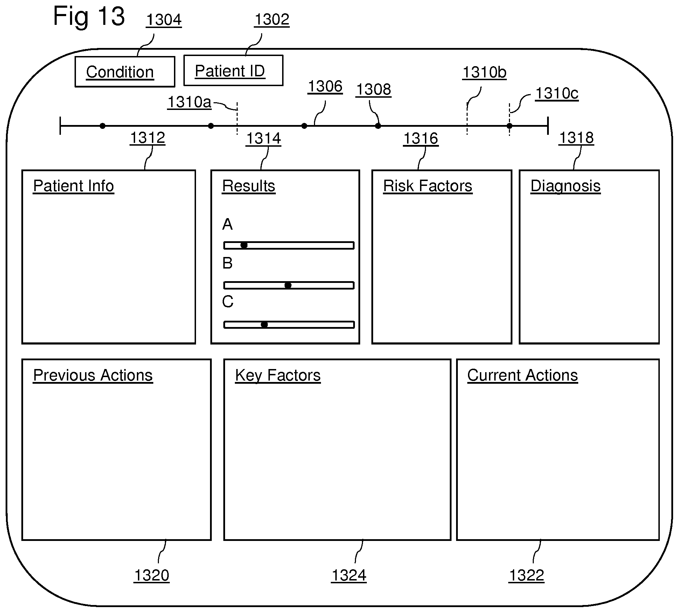

In an example set forth in Figure 13, the user interface may display a current status of a patient based on the determined status of at least one node and at least one edge in a selected directed graph using a patient model associated with the patient. As in previous examples, the user interface may display information relating to the Patient ID at block 1302 and information relating to the identified medical condition at block 1304. The user interface may similarly display a timeline 1306 summarising previous clinical steps in the patient history. For example, node 1308 may represent a previous clinical step such as a diagnostic scan. Lines 1310a-c may indicate important dates in the patient history.

The current patient status may be summarised by blocks 1312-1324 displaying respectively: patient information 1312, results relating to at least one clinical step 1314, risk factors related to a current treatment phase 1316, diagnostic information 1318, information summarising previous clinical steps 1320, information summarising current clinical steps 1322, and a summary of key factors 1324, which may be altered by a user, used in determining recommended treatments for the patient.

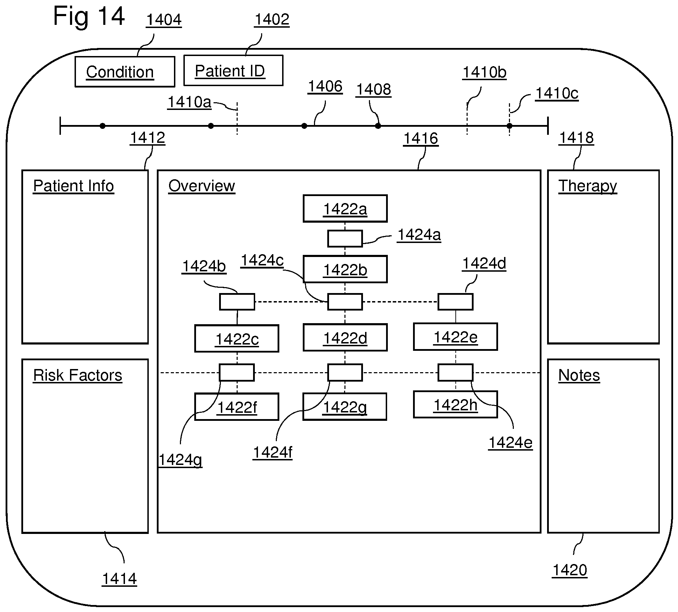

In an example set forth in Figure 14, the user interface may display an overview of the decision points along the clinical pathway. As in previous examples, the user interface may display information relating to the patient ID at block 1402 and information relating to the identified medical condition at block 1404. The user interface may display a timeline 1406 summarising previous clinical steps in the patient history. For example, node 1408 may represent a previous clinical step such as a diagnostic scan. Lines 1410a-c may indicate important dates in the patient history. Information relating to the patient may be displayed at block 1412, for example, the name, date of birth, body mass index, diagnosis, gender, whether the patient is a smoker etc. Risk factors associated with the patient may be displayed at block 1414, for example, comorbidities such as fibrosis, hypertension, emphysema, etc., and any medication the patient is taking.

At block 1416 an overview of the previous and/or current clinical steps may be shown as a decision graph. At block 1418 any recommended, current, or previous therapies may be described. At block 1430 any notes related to clinical steps or to the patient may be entered and/or review by a user of the user device.

Returning to block 1416, the decision graph may show an overview of the clinical pathway. Blocks 1422a-h may represent clinical steps and blocks 1424a-g may represent conditions for moving from one clinical step to another clinical step. For example, block 1422a may represent a determination of the cancer type, a test is performed and following the results 1424a the stage of the cancer may be determined at 1422b. Depending on the stage of the cancer different clinical steps may be prescribed for example, the cancer may be one of three stages indicated by 1424a-b. Depending which stage the cancer is the prescribed clinical step may be indicated by one of 1422c-e respectively.

The above embodiments may be used in a case in which a patient has a confirmed case of prostate cancer, for example. When analysing medical information associated with this patient, the system 100 may transit data indicative of a directed graph representing at least part of a relevant medical guideline to the user device 106a-d. For example, the system 100 may transmit data indicative of a directed graph representing at least part of the NCCN Prostate Cancer guideline or the EAU Prostate Cancer guideline. Tests prescribed in the guidelines may include blood tests to check for Prostate-Specific Antigen (PSA) levels. Other tests may include Prostate MRI scans to measure the size of the prostate and using a trans rectal ultrasound to measure the PSA density. Patient attributes which are measured and stored in a patient model in the case of prostate cancer may include PSA level, TNM classification, risk group assessment, PSA failure, biochemical failure, metastasis, disease progression. These patient attributes and their values may be used to stage a patient at a treatment phase of a medical guideline, for example by determining the statuses of nodes and directed edges in the directed graphs. As discussed above, during the treatment of a patient, there may be multiple options for treating a patient at a given time. Some of these options may be displayed as "recommended" for example those shown with check marks for example, 902g, 902t, 902s (Figure 9), 1106 (Figure 11), and options 1, 3, and 5 of 1218 (Figure 12). Some options may be shown as not recommended such as 1110 (Figure 11), and options 2 and 4 of 1218 (Figure 12). Other options may be undecidable, for example due to a lack of data in the patient model, shown at 902r and 902n (Figure 9), and 1112 (Figure 11). An example of such options is given for the case of prostate cancer below. For a patient with confirmed prostate cancer and a life expectancy of more than or equal to (≥) 10 years with an intermediate risk assessment, the potential options may include: Radical Prostatectomy without Pelvic Lymph-Node Dissection, Radical Prostatectomy with Pelvic Lymph-Node Dissection, Observation, or a treatment such as EBRT, ADT, or Brachytherapy.

The system 100 may determine the status of a node such as the node representing Radical Prostatectomy with Pelvic Lymph-Node Dissection. If the probability for Lymph-Node involvement for the patient being examined is ≥2%, then Radical Prostatectomy with Pelvic Lymph-Node Dissection is recommended. The likelihood of Lymph-Node involvement may be determined based on data stored in the patient model or alternatively, a user of the user device 106a-d may be prompted to determine and input the likelihood of Lymph-Node involvement for the current patient. If the probability of Lymph-Node involvement is <2% then Radical Prostatectomy with Lymph-Node Dissection is not recommended. In this case the node representing the Radical Prostatectomy without Lymph-Node Dissection will be recommended. If the probability of Lymph-Node involvement is not known, then a Radical Prostatectomy with or without Pelvic Lymph-Node Dissection is undecidable according to the guideline.

In another example, the above embodiments may be used in a case in which a patient has a confirmed case of lung cancer. Some of the patient attributes which may be used to stage a patient in a medical guideline such as the NCCN Non-Small Cell Lung Cancer guideline may include: distant metastasis, TNM classification, risk assessment, type of nodules, whether there are positive or negative Mediastinal Nodes, whether the cancer is resectable or in-operable, whether the patient is symptomatic or asymptomatic, and whether there is metastasis.

In a further example, the above embodiments may be used in a case in which a patient has a confirmed case of breast cancer. Some of the patient attributes which may be used to stage a patient in a medical guideline, such as the NCCN Breast Cancer guideline, may include: whether the cancer is ER-positive or negative, PR-positive or negative, whether HER2-positive or HER2 negative, whether the cancer is ductal or lobular, staging assessment, whether treatment is to be invasive or non-invasive, the progression of the disease, metastasis of the disease.

In a still further example, the above embodiments may be used in a case in which a patient has a confirmed case of Coronary Artery Disease (CAD). Some of the patient attributes which may be used to stage a patient in a medical guideline, such as the ASC Coronary Artery Disease guideline or the ESC Coronary Artery Disease guideline, may include: the pretreatment probability of outcome, the risk stratification, and a chest pain assessment.

The above examples are given for illustrative purposes. The systems described above may be used in the staging, and management of any patient having a disease for which at least one medical guideline is available.
Features relating to an example of the user interface may be used in combination with features of any other examples of the user interface.

### Numbered Clauses

The following numbered clauses describe various embodiments of the present invention.
1. A system operable to transmit healthcare data to a user device, the user device being configured for use in analysing medical information, the system comprising at least one processor and at least one memory including computer program code, the at least one memory and computer program code configured to, with the at least one processor, cause the system to perform the steps of:
   maintaining, in a database, data representing a first directed graph representing at least part of a first version of a medical guideline, the first directed graph comprising a first plurality of nodes and a first set of directed edges, each node of the first plurality of nodes being connected to at least one further node of the first plurality of nodes by one of the first set of directed edges, the first directed graph comprising a primary node and terminating in at least one end node;
   responsive to a determination that a second version of the medical guideline is available, generating a first set of associations, each being between one of a second, different, plurality of nodes of a directed graph that may represent at least part of the second version and a respective part of the second version;
   identifying, based on the first set of associations, one or more differences between the directed graph that may represent at least part of the second version and the first directed graph;
   generating a second directed graph dependent at least on the one or more differences and the first directed graph; and
   transmitting data representing the second directed graph for receipt by the user device.
2. The system of clause 1, wherein the determination is performed by:
   comparing metadata associated with the first medical guideline with metadata associated with one or more further, different, medical guidelines stored at one or more medical guideline repositories.
3. The system of clause 1 or clause 2, wherein each of the first plurality of nodes represents a clinical step.
4. The system of clause 3, wherein each of the first set of directed edges represents a conditional parameter value resulting from a said clinical step associated with one of the first plurality of nodes connected thereto.
5. The system of clause 4, wherein the at least one processor and at least one memory including computer program code are configured to, with the at least one processor, cause the system to perform the steps of:
   generating a second, different, set of associations, each being between one of a second, different, set of directed edges of the directed graph that may represent at least part of the second version and a respective part of the second version;
   identifying, based on the second set of associations, one or more further differences between the second set of directed edges and the first set of directed edges; and
   generating the second directed graph dependent at least on the one or more further differences and the first directed graph.
6. The system of any preceding clause, wherein the at least one processor and at least one memory including computer program code are configured to, with the at least one processor, cause the system to perform the steps of:
   responsive to the determination, comparing a first textual excerpt of the first version of the medical guideline to a corresponding second textual excerpt of the second version of the medical guideline; and
   transmitting data representing a result of the comparison for receipt by the user device.
7. The system of any preceding clause, wherein generating the second directed graph comprises:
   based on a user input indicative of a decision in respect of at least one of the one or more differences, selectively using at least part of the first version or the second version to generate a respective part of the second directed graph.
8. The system of any preceding clause, wherein the first directed graph comprises data indicative of at least one local modification made by a user of the system.
9. The system of clause 8, wherein a part of the second directed graph is based on the at least one local modification.
10. The system of any preceding clause, wherein the at least one processor and at least one memory including computer program code are configured to, with the at least one processor, cause the system to perform the steps of:
   maintaining, in a database, a patient model comprising healthcare data associated with a patient; and
   determining, based on a combination of the first directed graph and the patient model, a first clinical pathway represented by at least some of the first plurality of nodes and at least one of the first set of directed edges.
11. The system of clause 10, wherein generating the second directed graph comprises:
   determining, based on a combination of the first clinical pathway and the one or more identified differences, a second clinical pathway represented by at least some of a third, different, plurality of nodes comprised in the second directed graph and at least one of a third, different, set of directed edges comprised in the second directed graph.
12. The system of clause 11, wherein determining the second clinical pathway comprises:
   transmitting data indicative of a comparison between the first clinical pathway and the one or more differences;
   receiving, from the user device, data indicating a command; and
   determining the second clinical pathway based at least on the received command.
13. A method of transmitting healthcare data to a user device, the user device being configured for use in analysing medical information, the method comprising:
   maintaining, in a database, data representing a first directed graph representing at least part of a first version of a medical guideline, the first directed graph comprising a first plurality of nodes and a first set of directed edges, each node of the plurality of nodes being connected to at least one further node of the plurality of nodes by one of the set of directed edges, the first directed graph comprising a primary node and terminating in at least one end node;
   responsive to a determination that a second version of the medical guideline is available, generating a first set of associations, each being between one of a second, different, plurality of nodes of a directed graph that may represent at least part of the second version and a respective part of the second version;
   identifying, based on the first set of associations, one or more differences between the directed graph that may represent at least part of the second version and the first directed graph;
   generating a second directed graph based at least one the one or more differences and the first directed graph; and
   transmitting data representing the second directed graph for receipt by the user device.
14. A computer program comprising a set of instructions, which, when executed by a computerised device, cause the computerised device to perform a method of transmitting healthcare data to a user device, the user device being configured for use in analysing medical information, the method comprising, at the computerised device:
   maintaining, in a database, data representing a first directed graph representing at least part of a first version of a medical guideline, the first directed graph comprising a first plurality of nodes and a first set of directed edges, each node of the plurality of nodes being connected to at least one further node of the plurality of nodes by one of the set of directed edges, the first directed graph comprising a primary node and terminating in at least one end node;
   responsive to a determination that a second version of the medical guideline is available, generating a first set of associations, each being between one of a second, different, plurality of nodes of a directed graph that may represent at least part of the second version and a respective part of the second version;
   identifying, based on the first set of associations, one or more differences between the directed graph that may represent at least part of the second version and the first directed graph;
   generating a second directed graph based at least on the one or more differences and the first directed graph; and
   transmitting data representing the second directed graph for receipt by the user device.
15. A system operable to transmit healthcare data to a user device, the user device being configured for use in analysing medical information, the system comprising at least one processor and at least one memory including computer program code, the at least one memory and the computer program code configured to, with the at least one processor, cause the system to perform the steps of:
   maintaining, in a database, a plurality of directed graphs, each directed graph representing at least part of at least one medical guideline and comprising a plurality of nodes and a set of directed edges, each node of the plurality of nodes being connected to at least one further node of the plurality of nodes by one of the set of directed edges, each directed graph comprising a primary node and terminating in at least one end node;
   receiving, from the user device, healthcare data comprising data identifying a medical condition;
   determining a context parameter based on the received healthcare data and an identifier of the user device;
   selecting, based on the determined context parameter and the data identifying the medical condition, a directed graph from the plurality of directed graphs; and
   transmitting data indicative of the selected directed graph for receipt by the user device.
16. The system of clause 15, wherein each of the nodes represents a clinical step.
17. The system of clause 15 or clause 16, wherein each of the directed edges represents a conditional parameter value resulting from a said clinical step associated with one of the nodes connected thereto.
18. The system of any one of clauses 15 to 17, wherein the at least one processor and at least one memory including computer program code are configured to, with the at least one processor, cause the system to perform the step of:
   maintaining, in a database, a plurality of patient models, each comprising healthcare data associated with a respective patient.
19. The system of clause 18, wherein the context parameter comprises data indicative of one of the pluralities of patient models.
20. The system of clause 18 or clause 19, wherein the context parameter comprises data associated with at least one patient entry.
21. The system of any one of clauses 15 to 20, wherein the at least one processor and at least one memory including computer program code are configured to, with the at least one processor, cause the system to perform the steps of:
   selecting, based on the context parameter and the data identifying the medical condition, a further directed graph from the plurality of directed graphs; and
   transmitting data indicative of the selected further directed graph for receipt by the user device.
22. The system of any one of clauses 15 to 21, wherein the healthcare data comprising data identifying a medical condition received from the user device is received via a network interface over a wide area network.
23. The system of any one of clauses 15 to 22, wherein the context parameter comprises an indication of a location of the user device.
24. The system of any one of clauses 15 to 23, wherein the context parameter comprises an indication of a medical practitioner using the user device.
25. The system of any one of clauses 15 to 24, wherein the selected directed graph comprises data indicative of a local modification of at least one of the nodes and/or at least one of the set of directed edges of the selected directed graph.
26. The system of any one of clauses 15 to 25, wherein the at least one processor and at least one memory including computer program code are configured to, with the at least one processor, cause the system to perform the steps of:
   maintaining, in a database, a plurality of textual excerpts from the at least one medical guideline;
   selecting, based on the medical guideline represented by the selected directed graph, at least one textual excerpt from the plurality of textual excerpts; and
   transmitting data indicative of the textual excerpt for receipt by the user device.
27. The system of any one of clauses 15 to 26, wherein the system transmits data to the user device over a wide area network via a network interface.
28. A method of transmitting healthcare data to a user device, the user device being configured for use in analysing medical information, the method comprising:
   maintaining, in a database, a plurality of directed graphs, each directed graph representing at least part of at least one medical guideline and comprising a plurality of nodes and a set of directed edges, each node of the plurality of nodes being connected to at least one further node of the plurality of nodes by one of the set of directed edges, each directed graph comprising a primary node and terminating in at least one end node;
   receiving, from the user device, healthcare data comprising data identifying a medical condition;
   determining a context parameter based on the received healthcare data and an identifier of the user device;
   selecting, based on the determined parameter and the data identifying the medical condition, a directed graph from the plurality of directed graphs; and
   transmitting data indicative of the selected directed graph for receipt by the user device.
29. A computer program comprising a set of instructions, which, when executed by a computerised device, cause the computerised device to perform a method of transmitting healthcare data to a user device, the user device being configured for use in analysing medical information, the method comprising, at the computerised device:
   maintaining, in a database, a plurality of directed graphs, each directed graph representing at least part of at least one medical guideline and comprising a plurality of nodes and a set of directed edges, each node of the plurality of nodes being connected to at least one further node of the plurality of nodes by one of the set of directed edges, each directed graph comprising a primary node and terminating in at least one end node;
   receiving, from the user device, healthcare data comprising data identifying a medical condition;
   determining a context parameter based on the received healthcare data and an identifier of the user device;
   selecting, based on the determined parameter and the data identifying the medical condition, a directed graph from the plurality of directed graphs; and
   transmitting data indicative of the selected directed graph for receipt by the user device.
30. A system operable to transmit healthcare data to a user device, the user device being configured for use in analysing medical information, the system comprising at least one processor and at least one memory including computer program code configured to, with the at least one processor, cause the system at least to perform the steps of:
   maintaining, in a database, data representing a plurality of directed graphs, each directed graph representing at least part of at least one medical guideline and comprising a plurality of nodes and a set of directed edges, each node of the plurality of nodes being connected to at least one further node of the plurality of nodes by one of the set of directed edges, each directed graph comprising a primary node and terminating in at least one end node;
   maintaining, in a database, a plurality of patient models each comprising healthcare data associated with a respective patient;
   receiving, from the user device, healthcare data comprising data identifying a patient and a medical condition;
   selecting, based at least on the received data identifying the medical condition, a directed graph from the plurality of directed graphs;
   selecting, based on the received data identifying the patient, a patient model from the plurality of patient models;
   dependent on a combination of the selected directed graph and the selected patient model, retrieving further healthcare data; and
   transmitting the further healthcare data for receipt by the user device.
31. The system of clause 30, wherein the further healthcare data comprises at least one of:
   a textual excerpt from a said medical guideline associated with the selected directed graph;
   statistical information relating to a patient cohort associated with the selected patient model;
   a medical research paper; and
   consensus based supplementary medical data.
32. The system of clause 30 or clause 31, wherein each of the nodes represents a clinical step.
33. The system of clause 32, wherein each of the directed edges represents a conditional parameter value resulting from a said clinical step associated with one of the nodes connected thereto.
34. The system of clause 33, wherein the at least one processor and at least one computer memory including computer program code are configured to cause the system to perform the step of:
   maintaining, in a database, at least one association between the further healthcare data and at least one directed graph, and wherein the further healthcare data is retrieved according to the at least one association.
35. The system of clause 34, wherein the at least one association is between the further healthcare data and a said node, and wherein the further healthcare data is retrieved according to the at least one association.
36. The system of clause 35, wherein retrieving the further healthcare data is dependent on a status of at least one of the plurality of nodes and/or at least one of the set of directed edges of the selected directed graph.
37. The system of clause 36, wherein the status of a said node is dependent upon availability of data associated with a clinical step represented by the node.
38. The system of clause 36 or clause 37, wherein the status of a said directed edge is dependent upon a combination of data associated with a clinical step represented by a said node connected thereto and a conditional parameter value represented by the directed edge.
39. The system of any one of clauses 30 to 38, wherein the selected patient model comprises a plurality of patient entries and wherein determining a status of a said node and a said directed edge connected thereto comprises the steps of:
   maintaining a first association between at least one of the patient entries and an identifier from a plurality of identifiers;
   maintaining a second association between at least one of the nodes and an identifier from the plurality of identifiers;
   selecting, based on the first and second associations, a said attribute value associated with the node; and
   determining, based on a comparison of the attribute value associated with the node to a conditional parameter value represented by the directed edge, whether the conditional parameter value represented by the directed edge is satisfied.
40. The system of clause any one of clauses 30 to 39, wherein retrieving further healthcare data is dependent on a determination that a further, different, said directed graph based on the selected patient model does not correspond with the selected directed graph.
41. The system of clause 39, wherein retrieving further healthcare data is dependent on a determination that the status of at least one of the nodes and/or at least one of the set of directed edges of the selected directed graph cannot be determined based on the selected patient model.
42. The system of any one of clauses 30 to 41, wherein the at least one processor and at least one computer memory including computer program code are configured to cause the system to perform the steps of:
   maintaining, in a database, an association between a plurality of state parameters and the further healthcare data, the state parameters being for use in processing a decision at a said node of a directed graph;
   receiving, from the user device, data indicating a rating associated with the retrieved further healthcare data; and
   processing the rating to modify a said state parameter based on the received rating.
43. A computer program comprising a set of instructions, which, when executed by a computerised device, cause the computerised device to perform a method of transmitting healthcare data to a user device, the user device being configured for use in analysing medical information, the method comprising, at the computerised device:
   maintaining, in a database, data representing a plurality of directed graphs, each directed graph representing at least part of at least one medical guideline and comprising a plurality of nodes and a set of directed edges, each node of the plurality of nodes being connected to at least one further node of the plurality of nodes by one of the set of directed edges, each directed graph comprising a primary node and terminating in at least one end node;
   maintaining, in a database, a plurality of patient models each comprising healthcare data associated with a respective patient;
   receiving, from the user device, healthcare data comprising data identifying a patient and a medical condition;
   selecting, based at least on the received data identifying the medical condition, a directed graph from the plurality of directed graphs;
   selecting, based on the received data identifying the patient, a patient model from the plurality of patient models;
   dependent on a combination of the selected directed graph and the selected patient model, retrieving further healthcare data; and
   transmitting the further healthcare data for receipt by the user device.
44. A method of transmitting healthcare data to a user device, the user device being configured for use in analysing medical information associated with a patient, the method comprising:
   maintaining, in a database, data representing a plurality of directed graphs, each directed graph representing at least part of at least one medical and comprising a plurality of nodes and a set of directed edges, each node of the plurality of nodes being connected to at least one further node of the plurality of nodes by one of the set of directed edges, each directed graph comprising a primary node and terminating in at least one end node;
   maintaining, in a database, a plurality of patient models each comprising healthcare data associated with a respective patient;
   receiving, from the user device, healthcare data comprising data identifying a patient and a medical condition;
   selecting, based at least on the received data identifying the medical condition, a directed graph from the plurality of directed graphs;
   selecting, based on the received data identifying the patient, a patient model from the plurality of patient models;
   dependent on a combination of the selected directed graph and the selected patient model, retrieving further healthcare data; and
   transmitting the further healthcare data for receipt by the user device.
45. A system operable to transmit healthcare data to a user device, the user device being configured for use in analysing medical information, the system comprising at least one processor and at least one memory including computer program code, the at least one memory and computer program code configured to, with the at least one processor, cause the system to perform the steps of:
   maintaining, in a database, data representing a plurality of directed graphs, each directed graph representing at least part of at least one medical guideline and comprising a plurality of nodes and a set of directed edges, each node of the plurality of nodes being connected to at least one further node of the plurality of nodes by one of the set of directed edges, each directed graph comprising a primary node and terminating in at least one end node;
   maintaining, in a database, a plurality of patient models each comprising healthcare data associated with a respective patient;
   receiving, from the user device, healthcare data comprising data identifying a patient and a medical condition;
   selecting, based at least on the received data identifying the medical condition, a directed graph from the plurality of directed graphs;
   selecting, based on the received data identifying the patient, a patient model from the plurality of patient models;
   identifying, based on the selected patient model and the received healthcare data, a status of at least one of the nodes and at least one of the set of directed edges of the selected directed graph; and
   transmitting data associated with the status of the at least one of the nodes and the status of the at least one of the set of directed edges for receipt by the user device.
46. The system of clause 45, wherein each of the nodes represents a clinical step.
47. The system of clause 46, wherein each of the directed edges represents a conditional parameter value resulting from a said clinical step associated with one of the nodes connected thereto.
48. The system of clause 46 or clause 47, wherein the status of a said node is dependent upon availability of data associated with a said clinical step represented by the node.
49. The system of clause 47 or clause 48, wherein the status of a said directed edge is dependent upon a combination of data associated with a clinical step represented by a said node connected thereto and a conditional parameter value represented by the directed edge.
50. The system of clause 45, wherein the selected patient model comprises a plurality of patient entries and wherein determining a status of a said node and a said directed edge connected thereto comprises the steps of:
   maintaining a first association between at least one of the patient entries and an identifier from a plurality of identifiers;
   maintaining a second association between at least one of the nodes and an identifier from the plurality of identifiers;
   selecting, based on the first and second associations, a said attribute value associated with the node; and
   determining, based on a comparison of the attribute value associated with the node to the conditional parameter value represented by the directed edge, whether the conditional parameter value represented by the directed edge is satisfied.
51. The system of any one of clauses 45 to 50, wherein the at least one processor and at least one memory including computer program code are configured to, with the at least one processor, cause the system to perform the steps of:
   determining, based on the identified status of the at least one of the nodes and the at least one directed edge of the selected directed graph, whether a further, different, said directed graph based on the selected patient model corresponds with the selected directed graph; and
   dependent on the determination, transmitting data indicating the determination for receipt by the user device.
52. The system of clause any one of clauses 45 to 51, wherein the at least one processor and at least one memory including computer program code are configured to, with the at least one processor, cause the system to perform the steps of:
   determining, based on the identified status of the at least one of the nodes and the at least one directed edge, a start date and an end date of at least one treatment phase associated with the at least one medical guideline; and
   transmitting data indicating the start and the end date of the at least one treatment phase for receipt by the user device.
53. The system of clause 52, wherein the at least one processor and at least one memory including computer program code are configured to, with the at least one processor, cause the system to perform the steps of:
   retrieving further healthcare data associated with the at least one treatment phase; and
   transmitting the further healthcare data associated with the at least one treatment phase for receipt by the user device.
54. The system of clause 52 or clause 53, wherein the at least one treatment phase is represented in the selected directed graph by at least some of the plurality of nodes connected by at least one of the set of directed edges.
55. The system of any one of clauses 52 to 54, wherein the at least one processor and at least one memory including computer program code are configured to, with the at least one processor, cause the system to perform the steps of:
   identifying, for the at least one treatment phase, at least one node and at least one directed edge for which an attribute value associated cannot be selected based on the identifier; and
   determining the status of the at least one node and the at least one directed edge using the selected patient model and further healthcare data.
56. The system of clause any one of clauses 45 to 55, wherein the at least one processor and at least one memory including computer program code are configured to, with the at least one processor, cause the system to perform the steps of:
   transmitting data indicating the at least one node and the at least one directed edge for which an
      attribute value cannot be selected based on the identifier for receipt by the user device; and
   receiving, from the user device, data indicating the status of the at least one node and the at least one directed edge for which a said patient attribute value cannot be selected based on the identifier.
57. A method of transmitting healthcare data to a user device, the user device being configured for use in analysing medical information, the method comprising:
   maintaining, in a database, data representing a plurality of directed graphs, each directed graph representing at least part of at least one medical guideline and comprising a plurality of nodes and a set of direct edges, each node of the plurality of nodes being connected to at least one further node of the plurality of nodes by a directed edge, each directed graph comprising a primary node and terminating in at least one end node:
   maintaining, in a database, a plurality of patient models each comprising healthcare data associated with a respective patient;
   receiving, from the user device, healthcare data comprising data identifying a patient and a medical condition;
   selecting, based at least one the received data identifying the medical condition, a directed graph from the plurality of directed graphs;
   selecting, based on the received data identifying the patient, a patient model from the plurality of patient models;
   identifying, based on the selected patient model and the received healthcare data, a status of at least one of the nodes and at least one directed edge of the selected directed graph; and
   transmitting data associated with the status of the at least one of the nodes and the status of the at least one directed edge for receipt by the user device.
58. A computer program comprising a set of instructions, which, when executed by a computerised device, cause the computerised device to perform a method of transmitting healthcare data to a user device, the user device being configured for use in analysing medical information, the method comprising, at the computerised device:
   maintaining, in a database, data representing a plurality of directed graphs, each directed graph representing at least part of at least one medical guideline and comprising a plurality of nodes and a set of directed edges, each node of the plurality of nodes being connected to at least one further node of the plurality of nodes by one of the set of directed edges, each directed graph comprising a primary node and terminating in at least one end node;
   maintaining, in a database, a plurality of patient models each comprising healthcare data associated with a respective patient;
   receiving, from the user device, healthcare data comprising data identifying a patient and a medical condition;
   selecting, based at least on the received data identifying the medical condition, a directed graph from the plurality of directed graphs;
   selecting, based on the received data identifying the patient, a patient model from the plurality of patient models;
   identifying, based on the selected patient model and the received healthcare data, a status of at least one of the nodes and at least one directed edge of the selected directed graph; and
   transmitting data associated with the status of the at least one of the nodes and the status of the at least one directed edge for receipt by the user device.

## Claims

1. A system (100) operable to transmit healthcare data to a user device (106a-d), the user device (106a-d) being configured for use in analysing medical information, the system (100) comprising at least one processor (102a-n) and at least one memory (104a-n) including computer program code, the at least one memory (104a-n) and computer program code configured to, with the at least one processor (102a-n), cause the system (100) to perform the steps of:
maintaining, in a database (108), data representing a first directed graph representing at least part of a first version of a medical guideline, the first directed graph comprising a first plurality of nodes and a first set of directed edges, each node of the first plurality of nodes being connected to at least one further node of the first plurality of nodes by one of the first set of directed edges, the first directed graph comprising a primary node and terminating in at least one end node;
responsive to a determination that a second version of the medical guideline is available, generating a first set of associations, each being between one of a second, different, plurality of nodes of a directed graph that may represent at least part of the second version and a respective part of the second version;
identifying, based on the first set of associations, one or more differences between the directed graph that may represent at least part of the second version and the first directed graph;
generating a second directed graph dependent at least on the one or more differences and the first directed graph; and
transmitting data representing the second directed graph for receipt by the user device (106a-d).

2. The system (100) of claim 1, wherein the determination is performed by:
comparing metadata associated with the first medical guideline with metadata associated with one or more further, different, medical guidelines stored at one or more medical guideline repositories.

3. The system (100) of claim 1 or claim 2, wherein each of the first plurality of nodes represents a clinical step.

4. The system (100) of claim 3, wherein each of the first set of directed edges represents a conditional parameter value resulting from a said clinical step associated with one of the first plurality of nodes connected thereto.

5. The system (100) of claim 4, wherein the at least one processor (102a-n) and at least one memory (104a-n) including computer program code are configured to, with the at least one processor (102a-n), cause the system (100) to perform the steps of:
generating a second, different, set of associations, each being between one of a second, different, set of directed edges of the directed graph that may represent at least part of the second version and a respective part of the second version;
identifying, based on the second set of associations, one or more further differences between the second set of directed edges and the first set of directed edges; and
generating the second directed graph dependent at least on the one or more further differences and the first directed graph.

6. The system (100) of any preceding claim, wherein the at least one processor (102a-n) and at least one memory (104a-n) including computer program code are configured to, with the at least one processor (102a-n), cause the system (100) to perform the steps of:
responsive to the determination, comparing a first textual excerpt of the first version of the medical guideline to a corresponding second textual excerpt of the second version of the medical guideline; and
transmitting data representing a result of the comparison for receipt by the user device (106a-d).

7. The system (100) of any preceding claim, wherein generating the second directed graph comprises:
based on a user input indicative of a decision in respect of at least one of the one or more differences, selectively using at least part of the first version or the second version to generate a respective part of the second directed graph.

8. The system (100) of any preceding claim, wherein the first directed graph comprises data indicative of at least one local modification made by a user of the system (100).

9. The system (100) of claim 8, wherein a part of the second directed graph is based on the at least one local modification.

10. The system (100) of any preceding claim, wherein the at least one processor (102a-n) and at least one memory (104a-n) including computer program code are configured to, with the at least one processor (102a-n), cause the system (100) to perform the steps of:
maintaining, in a database (108), a patient model comprising healthcare data associated with a patient; and
determining, based on a combination of the first directed graph and the patient model, a first clinical pathway represented by at least some of the first plurality of nodes and at least one of the first set of directed edges.

11. The system (100) of claim 10, wherein generating the second directed graph comprises:
determining, based on a combination of the first clinical pathway and the one or more identified differences, a second clinical pathway represented by at least some of a third, different, plurality of nodes comprised in the second directed graph and at least one of a third, different, set of directed edges comprised in the second directed graph.

12. The system (100) of claim 11, wherein determining the second clinical pathway comprises:
transmitting data indicative of a comparison between the first clinical pathway and the one or more differences;
receiving, from the user device (106a-d), data indicating a command; and
determining the second clinical pathway based at least on the received command.

13. A method of transmitting healthcare data to a user device (106a-d), the user device (106a-d) being configured for use in analysing medical information, the method comprising:
maintaining, in a database (108), data representing a first directed graph representing at least part of a first version of a medical guideline, the first directed graph comprising a first plurality of nodes and a first set of directed edges, each node of the plurality of nodes being connected to at least one further node of the plurality of nodes by one of the set of directed edges, the first directed graph comprising a primary node and terminating in at least one end node;
responsive to a determination that a second version of the medical guideline is available, generating a first set of associations, each being between one of a second, different, plurality of nodes of a directed graph that may represent at least part of the second version and a respective part of the second version;
identifying, based on the first set of associations, one or more differences between the directed graph that may represent at least part of the second version and the first directed graph;
generating a second directed graph based at least one the one or more differences and the first directed graph; and
transmitting data representing the second directed graph for receipt by the user device (106a-d).

14. A computer program comprising a set of instructions, which, when executed by a computerised device, cause the computerised device to perform a method of transmitting healthcare data to a user device (106a-d), the user device (106a-d) being configured for use in analysing medical information, the method comprising, at the computerised device:
maintaining, in a database (108), data representing a first directed graph representing at least part of a first version of a medical guideline, the first directed graph comprising a first plurality of nodes and a first set of directed edges, each node of the plurality of nodes being connected to at least one further node of the plurality of nodes by one of the set of directed edges, the first directed graph comprising a primary node and terminating in at least one end node;
responsive to a determination that a second version of the medical guideline is available, generating a first set of associations, each being between one of a second, different, plurality of nodes of a directed graph that may represent at least part of the second version and a respective part of the second version;
identifying, based on the first set of associations, one or more differences between the directed graph that may represent at least part of the second version and the first directed graph;
generating a second directed graph based at least on the one or more differences and the first directed graph; and
transmitting data representing the second directed graph for receipt by the user device (106a-d).
